(19) Europäisches Patentamt
European Patent Office
Office européen des brevets

(11) Publication number: **0 181 709**
**B1**

(12) **EUROPEAN PATENT SPECIFICATION**

(45) Date of publication of patent specification: **09.05.90**

(21) Application number: **85307449.0**

(22) Date of filing: **16.10.85**

(51) Int. Cl.[5]: **C 07 C 217/76,** C 07 D 295/08, A 61 K 31/135, A 61 K 31/395

(54) **Dichloroaniline Derivatives.**

(30) Priority: **17.10.84 GB 8426191**

(43) Date of publication of application: **21.05.86 Bulletin 86/21**

(45) Publication of the grant of the patent: **09.05.90 Bulletin 90/19**

(84) Designated Contracting States: **AT BE CH DE FR IT LI LU NL SE**

(56) References cited:
**EP-A-0 119 446**
**FR-A-2 210 414**
**GB-A-2 088 873**

**The file contains technical information submitted after the application was filed and not included in this specification**

(73) Proprietor: **GLAXO GROUP LIMITED**
**Clarges House 6-12 Clarges Street**
**London W1Y 8DH (GB)**

(72) Inventor: **Skidmore, Ian Frederick**
**2 Wendover Drive**
**Welwyn Hertfordshire (GB)**
Inventor: **Finch, Harry**
**19 Hensley Close**
**Hitchin Hertfordshire (GB)**
Inventor: **Naylor, Alan**
**35 Layston Park**
**Royston Hertfordshire (GB)**
Inventor: **Campbell, Ian Baxter**
**5 Penegrine House The Blanes**
**Ware Hertfordshire (GB)**
Inventor: **Lunts, Lawrence Henry Charles**
**10 Wormley West End**
**Broxbourne Hertfordshire (GB)**

(74) Representative: **Marchant, James Ian et al**
**Elkington and Fife Beacon House 113 Kingsway**
**London WC2B 6PP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 181 709 B1

## Description

This invention relates to dichloroaniline derivatives having a stimulant action at $\beta_2$-adrenoreceptors, to processes for their preparation, to pharmaceutical compositions containing them and to their use in medicine.

Dihaloaniline derivatives have previously been described as bronchodilators having stimulant activity at $\beta$-adrenoreceptors.

Thus British Patent Specification NO. 1178191 describes compounds of the general structure

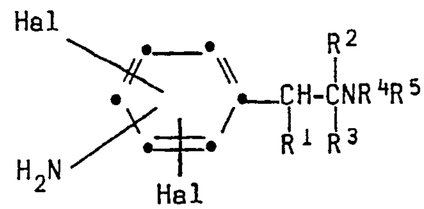

in which the substituents Hal represent bromine or chlorine atoms; $R^1$ represents hydrogen or hydroxyl; $R^2$ and $R^3$ each represent hydrogen or $C_{1-4}$alkyl; and $R^4$ and $R^5$ each represent hydrogen, $C_{1-6}$alkyl, alkenyl, alkynyl, hydroxyalkyl, alkoxyalkyl, dialkylaminoalkyl, cyclalkyl, phenyl, benzyl or adamantyl, or $NR^4R^5$ forms a heterocyclic ring optionally substituted by $C_{1-3}$alkyl groups.

French Patent Specification NO. 221041A describes compounds of the general structure

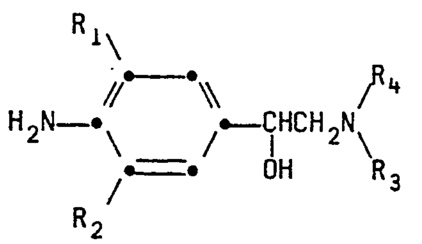

and

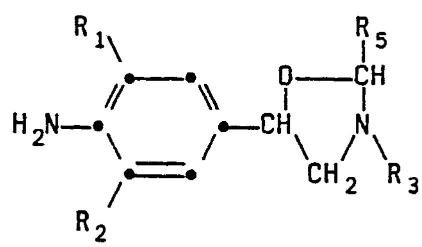

in which $R_1$ represents a hydrogen, fluorine, chlorine, bromine or iodine atom or a cyano group; $R_2$ represents a fluorine atom, an optionally branched $C_{1-5}$alkyl group or a hydroxyalkyl, aminoalkyl, dialkylaminoalkyl, trifluoromethyl, alkoxy, nitro, cyano, carboxy, carbalkoxy or carbamoyl group; $R_3$ and $R_4$ which may be the same or different each represent a hydrogen atom, an optionally branched $C_{1-6}$ alkyl group or a hydroxyalkyl, cycloalkyl, cycloalkylalkyl, alkenyl, alkynyl or optionally substituted aralkyl group; and $R_5$ represents a hydrogen atom or an optionally branched alkyl group.

UK Patent Specification NO. 2088873-A describes compounds of the general structure

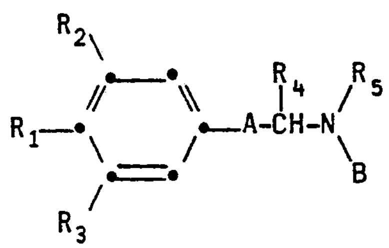

in which

$R_1$ represents a hydroxy group or an optionally substituted amino group; $R_2$ and $R_3$ which may be the same or different each represent a halogen atom, a trifluoromethyl, cyano or nitro group or one of $R_2$ and $R_3$ is a hydrogen atom;

$R_4$ represents a hydrogen atom or a $C_{1-3}$ alkyl group;

$R_5$ represents a hydrogen atom, an optionally branched $C_{1-4}$ alkyl group or a $C_{3-6}$ cycloalkyl, $C_{2-5}$ alkenyl or a $C_{7-10}$ aralkyl group;

A represents a methylene, ethylene or hydroxymethylene group; and B represents an optionally substituted aralkyl group in which the methylene adjacent to the phenyl ring may be replaced by an oxygen or sulphur atom or a sulphinyl or sulphonyl group.

European Patent Specification No. 0119446-A2 describes compounds of the general structure

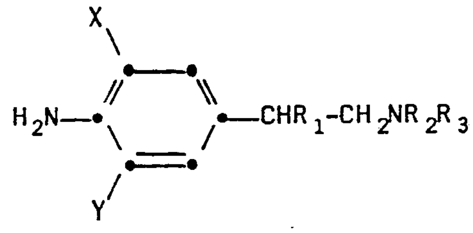

in which

X and Y which may be the same or different each represent a hydrogen or halogen atom;

$R_1$ represents a hydroxy group or a group $—OSir_4R_5R_6$;

$R_2$ represents a hydrogen atom or an optionally branched and optionally unsaturated $C_{1-6}$alkyl group (except that $R_2$ may only be alkyl when $R_3$ is $—SiR_4R_5R_6$);

$R_3$ represents an optionally branched and optionally unsaturated alkyl group substituted by a halogen atom; the group $—SiR_4R_5R_6$; or a phenyl group optionally substituted by a variety of substituents; or

$R_3$ represents a heterocyclic group or together with $R_2$ represents a nitrogen-containing heterocyclic ring; and

$R_4$, $R_5$ and $R_6$ each represent an optionally branched alkyl group.

We have now found a novel group of dichloroaniline derivatives, which differ structurally from those described in British Patent Specification No. 1178191, French Patent Specification No. 2210414-A, UK Patent Specification No. 0119446-A2, and which have a desirable and useful profile of activity.

Thus the present invention provides compounds of the general formula (I)

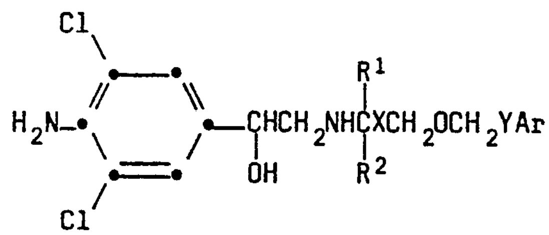

(I)

wherein

X represents a $C_{1-6}$ alkylene, $C_{2-6}$ alkenylene or $C_{2-6}$ alkynylene chain and

Y represents a bond, or a $C_{1-4}$ alkylene, $C_{2-4}$ alkenylene or $C_{2-4}$ alkynylene chain with the proviso that the sum total of carbon atoms in X and Y is not more than 8;

Ar represents a phenyl group optionally substituted by one or more substituents selected from halogen atoms, or the groups $C_{1-3}$ alkyl, $C_{1-3}$ alkoxy, $—(CH_2)_qOH$ (where q represents an integer from 0 to 3), $—NR^3R^4$ (where $R^3$ and $R^4$ each represents a hydrogen atom, or a $C_{1-4}$ alkyl group, or $NR^3R^4$ forms a saturated heterocyclic amino group which has 5—7 ring members and optionally contains in the ring one or more atoms selected from —O— or —S— or a group —NH— or $—N(CH_3)—$), or $—NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $—NR^3R^4$ group), or Ar is a phenyl group substituted by an alkylenedioxy group of formula $—O(CH_2)_pO—$, where p is 1 or 2;

$R^1$ and $R^2$ each represents a hydrogen atom or a $C_{1-3}$ alkyl group, with the proviso that the sum total of carbon atoms in $R^1$ and $R^2$ is not more than 4;

and physiologically acceptable salts and solvates (e.g hydrates) thereof.

It will be appreciated that the compounds of general formula (I) possess one or two asymmetric carbon atoms, namely the carbon atom of the

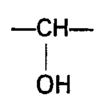

group and, when $R^1$ and $R^2$ are different groups, the carbon atom to which these are attached.

The compounds according to the invention thus include all enantiomers, diastereoisomers and mixtures thereof, including racemates. Compounds in which the carbon atom in the

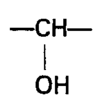

group is in the R configuration are preferred.

In the definition of general formula (I), the term alkenylene includes both *cis* and *trans* structures.

In one aspect, the invention provides compounds of formula (I) in which $R^1$, $R^2$, Ar and Y are as defined in formula (I) and X represents a $C_{2-6}$ alkylene, $C_{2-6}$ alkenylene or $C_{2-6}$ alkynylene chain.

In the general formula (I), the chain X may be for example $-(CH_2)_2-$, $-(CH_2)_3-$, $-(CH_2)_4-$, $-(CH_2)_5-$, $-(CH_2)_6-$, $-CH_2C{\equiv}C-$, $-(CH_2)_2CH{=}CH-$, $-(CH_2)_2C{\equiv}C-$, $-CH{=}CHCH_2-$, $-CH{=}CH(CH_2)_2-$ or $-CH_2C{\equiv}CCH_2-$. The chain Y may be for example $-CH_2-$, $-(CH_2)_2-$, $-(CH_2)_3-$, $-(CH_2)_4-$, $-CH{=}CH-$, $-C{\equiv}C-$, $CH_2CH{=}CH-$, or $-CH_2C{\equiv}C-$.

Preferably the total number of carbon atoms in the chains X and Y is 4 to 8 inclusive. Compounds wherein the sum total of carbon atoms in the chains X and Y is 4, 5, 6 or 7 are particularly preferred.

In one preferred group of compounds of formula (I) X represents a $C_{2-6}$ alkynylene or, more preferably, a $C_{2-6}$ alkylene chain and Y represents a $C_{1-4}$ alkylene chain. Particular compounds of this type are those wherein X is $-(CH_2)_3-$ or $-(CH_2)_4-$ and Y is $-CH_2-$, $-(CH_2)_2-$ or $-(CH_2)_3-$, or X is $-CH_2{\equiv}C-$ and Y is $-CH_2-$.

In the compounds of formula (I) $R^1$ and $R^2$ may each be, for example, methyl, ethyl, propyl or isopropyl groups except that if one of $R^1$ and $R^2$ is a propyl or isopropyl group, the other is a hydrogen atom or a methyl group. Thus for example $R^1$ may be a hydrogen atom or a methyl, ethyl or propyl group. $R^2$ may be, for example, a hydrogen atom or a methyl group. $R^1$ and $R^2$ are each preferably a hydrogen atom or a methyl group.

A preferred group of compounds are those wherein $R^1$ and $R^2$ are both hydrogen atoms, or $R^1$ is a hydrogen atom and $R^2$ is a $C_{1-3}$ alkyl group, particularly a methyl group.

When $-NR^3R^4$ in compounds of formula (I) represents a saturated heterocyclic amino group, This may have 5, 6 or 7 ring members and optionally contains in the ring a heteroatom selected from $-O-$ or $-S-$, or a group $-NH-$ or $-N(CH_3)-$. Examples of such $-NR^3R^4$ groups are pyrrolidino, piperidino, hexamethyleneimino, piperazino, N-methylpiperazino, morpholino, homomorpholino or thiamorpholino.

Ar may be for example a phenyl group. Examples of the optional substituents which may be present on the phenyl group represented by Ar include bromine, iodine, chlorine, fluorine, methyl, ethyl, methoxy, ethoxy, amino, methylamino, ethylamino, dimethylamino, diethylamino, morpholino, piperidino, piperazino, N-methylpiperazino, $-NHCOR^6$ [where $R^6$ is hydrogen, $C_{1-4}$ alkyl, (e.g. methyl, ethyl, isopropyl or n-butyl), $C_{1-4}$ alkoxy (e.g. methoxy, ethoxy, isopropoxy or n-butoxy), phenyl or amino], hydroxyl, $-CH_2OH$, or $-(CH_2)_2OH$.

The phenyl group represented by Ar may optionally contain one, two or three substituents, which may be present at the 2-, 3-, 4-, 5- or 6-positions on the phenyl ring.

Particular examples of a disubstituted phenyl group represented by Ar include phenyl substituted by two hydroxyl groups [e.g 3,5-dihydroxyphenyl], or a hydroxyl and a methoxy group [e.g. 3-methoxy-4-hydroxyphenyl].

Particular examples of a trisubstituted phenyl group represented by Ar include phenyl substituted by an amino and two methyl groups [e.g. 3,5-dimethyl-4-aminophenyl], an amino group and two chlorine atoms [e.g. 3,5-dichloro-4-aminophenyl], or three methoxy groups [e.g. 3,4,5-trimethoxyphenyl].

A preferred group of compounds are those of the formula (Ia)

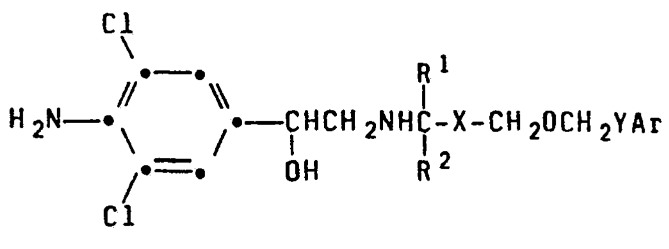

(Ia)

wherein

X represents a $C_{3-4}$ alkylene or $C_3$ alkynylene chain;

Y represents a $C_{1-3}$ alkylene chain;

$R^1$ and $R^2$ each represent hydrogen or methyl; and

Ar represents a phenyl group optionally substituted by a fluorine atom, a group selected from amino, $C_{1-3}$ alkyl (e.g. methyl), $C_{1-3}$ alkoxy (e.g. methoxy), hydroxy $C_{1-2}$ alkyl (e.g. hydroxymethyl), morpholino, hydroxy or $-NHCOR^6$ where $R^6$ is $C_{1-3}$ alkyl (e.g. methyl), or Ar is a phenyl group substituted by hydroxyl groups at the 3- and 5-positions; and physiologically acceptable salts and solvates thereof.

A particularly preferred group of compounds of formula (Ia) are those wherein Ar is a phenyl group optionally containing one substituent, more preferably an amino, —NHAcetyl or morpholino group.

Particularly important compounds of the invention are:

4-amino-3,5-dichloro-α-[[[6-[2-[4-(4-morpholinyl)phenyl]ethoxy]hexyl]amino]methyl]benzenemethanol;

N-[4-[2-[[6-[[2-(4-amino-3,5-dichlorophenyl)-2-hydroxyethyl]amino]hexyl]oxy]ethyl]phenyl]acetamide;

4-amino-3,5-dichloro-α-[[[6-[2-(4-aminophenyl)ethoxy]hexyl]amino]methyl]benzenemethanol;

4-amino-3,5-dichloro-α-[[[5-(2-phenylethoxy]pentyl]amino]methyl]benzenemethanol;

4-amino-3,5-dichloro-α-[[[6-(2-phenylethoxy)hexyl)]amino]methyl]benzenemethanol;
4-amino-3,5-dichloro-α-[[[1-methyl-5-(2-phenylethoxy)pentyl]amino]methyl]benzenemthanol;
4-amino-3,5-dichloro-α-[[[5-(2-phenylethoxy)-3-pentynyl]amino]methyl]benzenemethanol;
[3-[[5-[[2-(4-amino-3,5-dichlorophenyl)-2-hydroxyethyl]amino]pentyl]oxy]propyl]-1,3-benzenediol;
and the physiologically acceptable salts and solvates thereof.

Suitable physiologically acceptable salts of the compounds of general formula (I) include acid addition salts derived from inorganic and organic acids, such as hydrochlorides, hydrobromides, sulphates, phosphates, maleates, tartrates, citrates, benzoates, 4-methoxybenzoates, 2- or 4-hydroxybenzoates, 4-chlorobenzoates, p-toluenesulphonates, methanesulphonates, sulphamates, ascorbates, salicylates, acetates, fumarates, succinates, lactates, glutarates, gluconates, tricarballylates, hydroxy-naphthalene-carboxylates e.g. 1-hydroxy- or 3-hydroxy-2-naphthalenecarboxylates, or oleates. The compounds may also form salts with suitable bases. Examples of such salts are alkali metal (e.g. sodium and potassium), and alkaline earth metal (e.g. calcium or magnesium) salts.

The compounds according to the invention have a stimulant action at $\beta_2$-adrenoreceptors, which furthermore is of a particularly advantageous profile. The stimulant action was demonstrated in the isolated trachea of the guinea-pig, where compounds were shown to cause relaxation of PGF2α-induced contractions. Compounds according to the invention have shown a particularly long duration of action in this test.

The compounds according to the invention may be used in the treatment of diseases associated with reversible airways obstruction such as asthma and chronic bronchitis.

The compounds according to the invention are also indicated as useful for the treatment of inflammatory and allergic skin diseases, congestive heart failure, depression, premature labour, glaucoma, and in the treatment of conditions in which there is an advantage in lowering gastric acidity, particularly in gastric and peptic ulceration.

The invention accordingly further provides compounds of formula (I) and their physiologically acceptable salts and solvates for use in the therapy or prophylaxis of diseases associated with reversible airways obstruction in human or animal subjects.

The compounds according to the invention may be formulated for administration in any convenient way. The invention therefore includes within its scope pharmaceutical compositions comprising at least one compound of formula (I) or a physiologically acceptable salt or solvate thereof formulated for use in human or veterinary medicine. Such compositions may be presented for use with physiologically acceptable carriers or excipients, optionally with supplementary medicinal agents.

The compounds may be formulated in a form suitable for administration by inhalation or insufflation, or for oral, buccal, parenteral, topical (including nasal) or rectal administration. Administration by inhalation or insufflation is preferred.

For administration by inhalation the compounds according to the invention are conveniently delivered in the form of an aerosol spray presentation from pressurised packs, with the use of a suitable propellant, such as dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in for example capsules or cartridges of e.g. gelatin, or blister packs from which the powder may be administered with the aid of an inhaler or insufflator.

For oral administration, the pharmaceutical composition may take the form of, for example, tablets, capsules, powders, solutions, syrups or suspensions prepared by conventional means with acceptable excipients.

For buccal administration the composition may take the form of tablets, drops or lozenges formulated in conventional manner.

The compounds of the invention may be formulated for parenteral administration by bolus injection or continuous infusion. Formulations for injection may be presented in unit dosage form in ampoules, or in multi-dose containers with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water, before use.

For topical administration the pharmaceutical composition may take the form of ointments, lotions or creams formulated in a conventional manner, with for example an aqueous or oily base, generally with the addition of suitable thickening agents and/or solvents. For nasal application, the composition may take the form of a spray, formulated for example as an aqueous solution or suspension or as an aerosol with the use of a suitable propellant.

The compounds of the invention may also be formulated in rectal compositions such as suppositories or retention enemas, e.g. containing conventional suppository bases such as cocoa butter or other glyceride.

Where pharmaceutical compositions are described above for oral, buccal, rectal or topical

administration, these may be presented in a conventional manner associated with controlled release forms.

A proposed daily dosage of active compound for the treatment of man is 0.005 mg to 100 mg, which may be conveniently administered in one or two doses. The precise dose employed will of course depend on the age and condition of the patient and on the route of administration. Thus a suitable dose for administration by inhalation is 0.005 mg to 20 mg, for oral administration is 0.02 mg to 100 mg, and for parenteral administration is 0.01 mg to 2 mg for administration by bolus injection and 0.01 mg to 25 mg for administration by infusion.

The compounds according to the invention may be prepared by a number of processes, as described in the following wherein X, Y, Ar, $R^1$ and $R^2$ are as defined for general formula (I) unless otherwise specified. It will be appreciated that certain of the reactions described below are capable of affecting other groups in the starting material which are desired in the end product; this applies especially in the reduction processes described, particularly where hydrogen and a catalyst are used and when an ethylene or acetylene linkage is required in the compound of the invention. Care must therefore be taken in accordance with conventional practice, either to use reagents which will not affect such groups, or to perform the reaction as part of a sequence which avoids their use when such groups are present in the starting material.

In the preparation of both intermediates and end-products the final step in the reaction may be the removal of a protecting group. Conventional protecting groups may be used, as described for example in "Protective Groups in Organic Chemistry", Ed. J. F. W. McOmie (Plenum Press, 1973). Thus hydroxyl groups may for example be protected by aralkyl groups such as benzyl, diphenylmethyl or triphenylmethyl, or as tetrahydropyranyl derivatives. Suitable amino protecting groups include aralkyl groups such as benzyl, α-methylbenzyl, diphenylmethyl or triphenylmethyl, and acyl groups such as acetyl, trichloroacetyl or trifluoroacetyl.

Conventional methods of deprotection may be used. Thus for example aralkyl groups may be removed by hydrogenolysis in the presence of a metal catalyst (e.g. palladium on charcoal). Tetrahydropyranyl groups may be cleaved by hydrolysis under acidic conditions. Acyl groups may be removed by hydrolysis with a base such as sodium hydroxide or potassium carbonate, or a group such as trichloroacetyl may be removed by reduction with, for example, zinc and acetic acid.

In one general process (1), a compound of general formula (I) may be prepared by alkylation. Conventional alkylation procedures may be used.

Thus, for example, in one process (a), a compound of general formula (I) in which $R^1$ is a hydrogen atom may be prepared by alkylation of an amine of general formula (II)

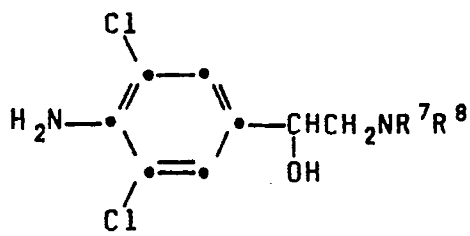

(II)

(wherein $R^7$ is a hydrogen atom or a protecting group and $R^8$ is a hydrogen atom) followed by removal of any protecting group where present.

The alkylation (a) may be effected using an alkylating agent of general formula (III):

$$\underset{\displaystyle R^2}{\underset{|}{LCH}}XCH_2OCH_2YAr \qquad \text{(III)}$$

(wherein L is a leaving group, for example a halogen atom such as chlorine, bromine or iodine, or a hydrocarbylsulphonyloxy such as methanesulphonyloxy or p-toluenesulphonloxy).

The alkylation is preferably effected in the presence of a suitable acid scavenger, for example, inorganic bases such as sodium or potassium carbonate, organic bases such as triethylamine, diisopropylethylamine or pyridine, or alkylene oxides such as ethylene oxide or propylene oxide. The reaction is conveniently effected as in a solvent such as acetonitrile or an ether e.g. tetrahydrofuran or dioxan, a ketone e.g. butanone or methyl isobutyl ketone, a substituted amide e.g. dimethylformamide or a chlorinated hydrocarbon e.g. chloroform at a temperature between ambient and the reflux temperature of the solvent.

According to another example (b) of an alkylation process, a compound of general formula (I) in which $R^1$ represents a hydrogen atom may be prepared by alkylation of an amine of general formula (II), as previously defined except that $R^8$ is a hydrogen atom or a group convertible thereto under the reaction conditions, with a compound of general formula (IV):

$R^2COXCH_2OCH_2YAr$ (IV)

in the presence of a reducing agent, followed when necessary by removal of any protecting groups.

Examples of suitable $R^8$ groups convertible into a hydrogen atom are arylmethyl groups such as benzyl, α-methylbenzyl and benzhydryl.

Suitable reducing agents include hydrogen in the presence of a catalyst such as platinum, platinum oxide, palladium oxide, Raney nickel or rhodium, on a support such as charcoal, using an alcohol, e.g. ethanol or methanol, or an ester e.g. ethyl acetate, or an ether e.g. tetrahydrofuran, or water, as reaction solvent, or a mixture of solvents, e.g. a mixture of two or more of those just described at normal or elevated temperature and pressure, for example from 20 to 100°C and from 1 to 10 atmospheres.

Alternatively when one or both of $R^7$ and $R^8$ are hydrogen atoms, the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols such as methanol or ethanol, or ethers such as diethyl ether or *tert*-buty methyl ether, or tetrahydrofuran.

When a compound of formula (II) where $R^7$ and $R^8$ are each hydrogen atoms is used, the intermediate imine of formula (V) may be formed:

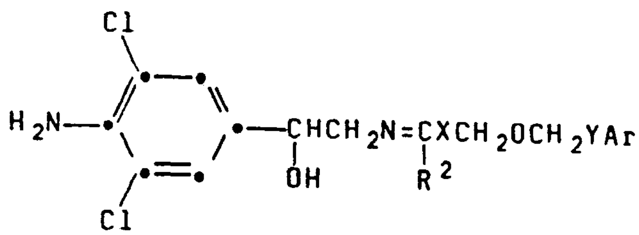

(V)

Reduction of the imine using the conditions described above, followed, where necessary, by removal of any protecting groups, gives a compound of general formula (I).

Where it is desired to use a protected intermediate of general formula (II) it is particularly convenient to use hydrogen and a catalyst as described above with protecting groups $R^7$ which are capable of being converted to a hydrogen atom under these reducing conditions, thus avoiding the need for a separate deprotection step. Suitable protecting groups of this type include arylmethyl groups such as benzyl, benzhydryl and α-methylbenzyl.

In another general process (2), a compound of general formula (I) may be prepared by reduction. Thus, for example, a compound of general formula (I) may be prepared by reducing an intermediate of general formula (VI):

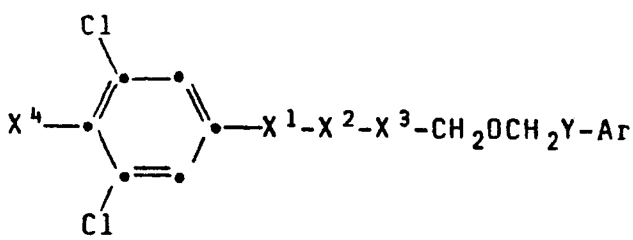

(VI)

wherein at least one of $X^4$, $X^1$, $X^2$, $X^3$ and Y represents a reducible group and/or Ar contains a reducible group and the other(s) take the appropriate meaning as follows, which is $X^4$ is $-NH_2$, $X^1$ is $-CH(OH)-$, $X^2$ is $-CH_2NR^7-$ and $X^3$ is $-CR^1R^2X$, followed where necessary by removal of any protecting groups.

Suitable reducible groups include those wherein $X^4$ is $-NO_2$, $X^1$ is a group $-C{=}O$, $X^2$ is a group $-CH_2NY'-$ (wherein Y′ represents a group convertible to hydrogen by catalytic hydrogenation, for example an arylmethyl group such as benzyl, benzhydryl or α-methylbenzyl), or an imine ($-CH{=}N-$) group or a group $-CONH-$, $X^3$ is a group $-COX-$ or a group $CR^1R^2X$ (where X is $C_{2-6}$ alkenylene or $C_{2-6}$ alkynylene), or $X^2-X^3-$ is a group $-CH_2N{=}CR^2X-$, Y is $C_{2-4}$ alkenylene or alkynylene, and Ar is a phenyl group substituted by a nitro group or by a group $-CHO$ or $-CO_2R^9$ where $R^9$ is hydrogen or an alkyl (e.g. $C_{1-3}$ alkyl) group.

The reduction may be effected using reducing agents conveniently employed for the reduction of carboxylic acids, aldehydes, esters, ketones, imines, amides, protected amines, alkenes, alkynes and nitro groups. Thus, for example, when $X^4$ in general formula (VI) represents a nitro group, or the phenyl group Ar contains a nitro substituent, this may be reduced to a $-NH_2$ group using hydrogen in the presence of a catalyst as previously described for process (1) part (b).

When $X^1$ in general formula (VI) represents a $-C{=}O$ group this may be reduced to a $-CH(OH)-$ group using hydrogen in the presence of a catalyst as previously described for process (1) part (b). Alternatively, the reducing agent may be, for example, a hydride such as diborane or a metal hydride such as lithium

aluminium hydride, sodium bis(2-methoxyethoxy) aluminium hydride, sodium borohydride or aluminium hydride. The reaction may be effected in a solvent, where appropriate an alcohol e.g. methanol or ethanol, or an ether such as tetrahydrofuran, or a halogenated hydrocarbon such as dichloromethane.

When $X^2$ in general formula (VI) represents a $—CH_2NY'—$ group or the group $—CH=N—$, or $—X^2—X^3—$ represents $—CH_2N=CR^2X—$ this may be reduced to a $—CH_2NH—$ or $—CH_2NHCHR^2X—$ group using hydrogen in the presence of a metal catalyst as previously described for process (1) part (b). Alternatively, when $X^2$ or $—X^2—X^3—$ is the group $—CH=N—$ or $—CH_2N=CR^2X—$ this may be reduced to a $—CH_2NH—$ or $—CH_2NHCHR^2X—$ group using a reducing agent and conditions as just described for the reduction of $X^1$ when this represents a $—C=O$ group.

When $X^2$ or $X^3$ in general formula (VI) represents a $—CONH—$ or $—COX—$ group this may be reduced to a group $—CH_2NH—$ or $—CH_2X—$ respectively using a hydride such as diborane or a complex metal hydride such as lithium aluminium hydride or sodium bis(2-methoxyethoxy)aluminium hydride in a solvent such as ether, e.g. tetrahydrofuran or diethyl ether.

When $X^3$ represents a group $CR^1R^2X$ where X is $C_{2-6}$ alkenylene or $C_{2-6}$ alkylylene, or Y represents $C_{2-4}$ alkenylene or $C_{2-4}$ alkynylene, this may be reduced to $C_{2-6}$ alkylene or $C_{2-4}$ alkylene respectively using hydrogen in the presence of a catalyst as previously described for process (1) part (b). Alternatively, when X is $C_{2-6}$ alkynylene or Y is $C_{2-4}$ alkynylene this may be reduced to $C_{2-4}$ alkenylene or $C_{2-4}$ alkenylene respectively using for example hydrogen and a lead-poisoned palladium on calcium carbonate catalyst in a solvent such as pyridine, or lithium aluminium hydride in a solvent such as diethyl ether at a low temperature e.g. 0°C.

When Ar is phenyl substituted by a group $—CHO$ or $—CO_2R^9$ where $R^9$ is hydrogen or alkyl this may be reduced to phenyl substituted by a hydroxymethyl group using for example a complex metal hydride such as lithium aluminium hydride or sodium borohydride.

In the general processes described above, the compound of formula (I) obtained may be in the form of a salt, conveniently in the form of a physiologically acceptable salt. Where desired, such salts may be converted to the corresponding free acids using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or iso-propanol.

Physiologically acceptable salts may also be prepared from other salts, including other physiologically acceptable salts, of the compounds of general formula (I), using conventional methods.

When a specific enantiomer of a compound of general formula (I) is required, this may be obtained by resolution of a corresponding racemate of a compound of general formula (I) using conventional methods.

Thus, in one example an appropriate optically active acid may be used to form salts with the racemate of a compound of general formula (I). The resulting mixture of isomeric salts may be separated for example by fractional crystallisation, into the diastereoisomeric salts from which the required enantiomer of a compound of general formula (I) may be isolated by conversion into the required free base.

Alternatively, enantiomers of a compound of general formula (I) may be synthesised from the appropriate optically active intermediates using any of the general processes described herein.

Specific diastereoisomers of a compound of formula (I) may be obtained by conventional methods for example, by synthesis from an appropriate asymmetric starting material using any of the processes described herein, or by conversion of a mixture of isomers of a compound of general formula (I) into appropriate diastereoisomeric derivatives e.g. salts which then can be separated by conventional means e.g. by fractional crystallisation.

Suitable methods for preparing the intermediate compounds used in the above general processes are described below. In the following discussion, Ar, $R^1$, $R^2$, $R^9$, $R^{10}$, $X^1$, $X^2$, $X^3$, $X^4$, Y, Y', and L are as defined above except where otherwise indicated. In addition, any substituent in the group Ar may be a precursor substituent which is convertible into the required substituent during the subsequent final-step process. "Hal" represents a halogen atom.

Intermediate compounds of general formula (VI) for use in general process (2) may be prepared by a number of processes.

Thus for example intermediates of general formula (VI) in which $X^1$ is a group $—C=O$ may be prepared from a haloketone of formula (VII)

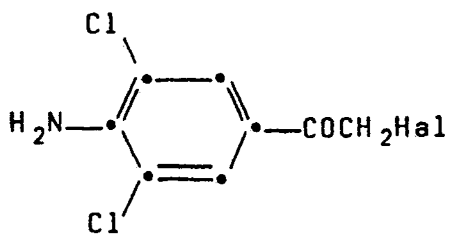

(VII)

by reaction with an amine of general formula (VIII)

$$\begin{array}{c} R^1 \\ | \\ Y'NHCXCH_2OCH_2YAr \\ | \\ R^2 \end{array} \qquad (III)$$

(wherein $Y^1$ is hydrogen or a group convertible thereto by catalytic hydrogenation). The reaction may be effected in a cold or hot solvent, for example tetrahydrofuran, *tert*-butyl methyl ether, dioxan, chloroform, dimethylformamide, acetonitrile or a ketone such as butanone or methylisobutylketone, or an ester, for example ethyl acetate, preferably in the presence of a base such as diisopropylethylamine, sodium carbonate or other acid scavenger such as propylene oxide.

Intermediates of general formula (VI) in which $X^1$ is a group —C=O may be reduced to the corresponding intermediate in which $X^1$ is a group —CH(OH)— using for example a metal hydride such as sodium borohydride in a solvent e.g. ethanol.

Iminoketones of general formula (VI) i.e. in which $X^2$ is a group —CH=N— may be obtained from a phenylglyoxal derivative of formula (IX):

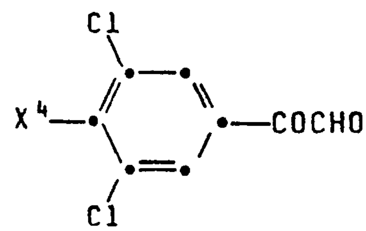

(IX)

by reaction with an amine of formula (VIII) in which Y' represents a hydrogen atom in a solvent such as benzene, tetrahydrofuran or an alcohol e.g. ethanol at temperatures up to the reflux. The phenylglyoxal derivatives of formula (IX) may be obtained from a haloketone of formula (VII) by the action of a dialkylsulphoxide such as dimethylsulphoxide.

Intermediates of general formula (VI) in which $X^3$ is a group —COX— may be prepared by acylation of an amine of formula (X):

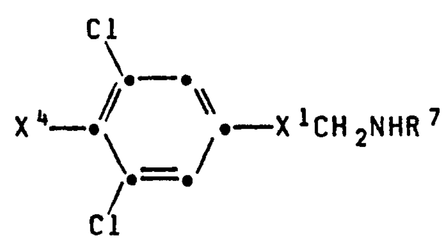

(X)

where $R^7$ is a hydrogen atom using an ester or an activated derivative of an acid of formula (XI):

$$ArYCH_2OCH_2XCO_2H \qquad (XI)$$

Suitable activated derivatives include the acid chloride, an anhydride or imidazolide. The reaction may be optionally carried out in a solvent such as tetrahydrofuran, benzene or chloroform, optionally in the presence of a base such as pyridine or triethylamine. The acids (XI) may be used directly if a coupling agent such as dicyclohexylcarbodiimide is added.

Acids of formula (XI) may be obtained by treatment of an alcohol of general formula (XII)

$$ArYCH_2OCH_2XCH_2OH \qquad (XII)$$

with a suitable oxidising agent, for example pyridinium dichromate in a solvent such as dimethylformamide.

Intermediates of formula (VI) in which $—X^2—X^3—$ represents $—CH_2N=CR^2X—$ may be obtained by reaction of an amine of formula (X) in which $R^7$ is a hydrogen atom with a compound of formula (IV) in a solvent such as acetonitrile.

Intermediates of formula (VI) in which $X^2$ is —CONH— may be prepared by reaction of an amine of formula (VIII) in which Y' is a hydrogen atom with an acid of formula (XIII):

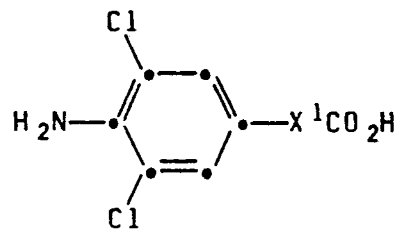

(XIII)

in the presence of a coupling agent such as dicyclohexylcarbodiimide. The acids of formula (XIII) may be prepared by methods analogous to conventional methods for the preparation of α-keto and α-hydroxycarboxylic acids.

Intermediates of formulae (II), (III), (IV), (VII), (VIII) and (XII) are either known compounds or may be prepared by methods analogous to those described for the preparation of known compounds.

Suitable methods for preparing intermediates of formulae (III), (IV), (VIII) and (XII) are described in UK Patent Specification No. 2140800A and in the exemplification included hereinafter.

In addition, for the preparation of ketones of formula (IV) (in which $R^2$ represents an alkyl group), a halide $ArYCH_2OCH_2XHal$ (where X represents a bond, $C_{1-5}$ alkylene, $C_{2-5}$ alkenylene or $C_{2-4}$ alkynylene) may be reacted with an appropriate β-ketoester or β-diketone under basic conditions to give an alkylated derivative, which on hydrolysis affords a ketone of formula (IV).

The following examples illustrate the invention. Temperatures are in °C. 'Dried' refers to drying using magnesium sulphate except where otherwise stated. Thin layer chromatography (t.l.c.) was carried out over $SiO_2$. Flash column chromatography (FCC) was carried out on silica (Merck 9385). The following abbreviations are used: THF — tetrahydrofuran; EA — ethyl acetate; ER — diethyl ether; CX — cyclohexane; H — hexane; DMF — dimethylformamide; DCM — dichloromethane; TE — triethylamine; ME — methanol; T — toluene; ET — ethanol.

### Intermediate 1

Referred to below is 1-(4-amino-3,5-dichlorophenyl)-2-bromo-1-ethanone.

### Intermediate 2

(a) *1-[2-[(6-Bromohexyl)oxy]ethyl]-4-nitrobenzene* 4-Nitrobenzeneethanol (10.25 g), 1,6-dibromohexane (27 ml) tetra-n-butylammonium bisulphate (1.7 g) and 12.5 M aqueous sodium hydroxide (55 ml) were stirred together for 40h. The mixture was diluted with water (250 ml), extracted with ER (3 × 350 ml) and the combined extracts were washed consecutively with water (250 ml) and brine (250 ml), dried and evaporated to give an oil (42.6 g). The oil was purified by FCC eluting with ER—CX (0:100 → 1:19) to give the *title compound* as a yellow oil (9.52 g). T.l.c. (ER—CX 1:19) Rf 0.11.

The following compounds were similarly prepared:

(b) *1-[2-[(5-Bromopentyl)oxy]ethyl]-4-methylbenzene* (17.9 g) as a colourless oil, from 4-methylbenzeneethanol (10.0 g) and 1,5-dibromopentane (50.7 g) with stirring at room temperature for 72h. T.l.c (ER—CX 1:19) Rf 0.29.

(c) *1-[3-[(5-Bromopentyl)oxy]propyl]-3,5-bis(phenylmethoxy)benzene* (4.98 g) as a colourless oil, from 3,5-bis(phenylmethoxy)benzenepropanol (5.0 g) and 1,5-dibromopentane (5.9 ml) with stirring at room temperature for 16h. T.l.c. (ER—CX 1:19) Rf 0.13.

### Intermediate 3

[2-[(5-Bromo-2-pentynyl)oxy]ethyl]benzene

(i) *[2-[(2-Propynyl)oxy]ethyl]benzene*

A mixture of benzenethanol (12.2 g), 3-bromo-1-propyne (12.0 ml), 40% aqueous sodium hydroxide (20 ml) and tetrabutylammonium bisulphate (1g) was stirred overnight. Water (100 ml) was added and the mixture was extracted with ER (2 × 100 ml). The organic extracts were washed with water and brine, dried and concentrated to a dark oil which was purified by FCC eluting with CX—ER (19:1) to give the *title compound* as a pale yellow oil (12.3 g) T.l.c (CX—ER 19:1) Rf 0.50.

(ii) *5-(2-Phenylethoxy)-3-pentyn-1-ol*

n-Butyl lithium (1.6M in hexane, 35 ml) was added to a stirred solution of the product of step (i) (8.0 g) in dry THF (50 ml) at −78° under nitrogen. Boron trifluoride etherate (6.8 ml) was added and the mixture was stirred at −78° for 30 min. Oxirane (7 ml) was added and the mixture was stirred at −78° for 1 h, treated with saturated saturated aqueous ammonium chloride (100 ml), allowed to warm to room temperature, and extracted with ER (2 × 100 ml). The organic extracts were washed with water and brine, dried and concentrated to an orange oil which was purified by FCC eluting with H—ER (2:1) to give the *title compound* as a pale yellow oil (3.95 g). T.l.c. (H—ER 2:1) Rf 0.10.

(iii) *[2-[(5-Bromo-2-pentynyl)oxy]ethyl]benzene*

A solution of triphenylphosphine (5.25 g) in dry DCM (15 ml) was added to a solution of the product of step (ii) (3.9 g) and carbon tetrabromide (6.93 g) in dry DCM (25 ml) at 0° over 10 min. The yellow solution was stirred at 0° for 30 min, evaporated onto silica (Merck 9385) and purified by FCC eluting with H → H—ER (3:1) to give the *title compound* as a colourless oil (2.7 g). T.l.c. (H—ER 2:1) Rf 0.69.

Intermediate 4
N-[1-Methyl-5-(2-phenylethoxy)pentyl]benzenemethanamine

(i) *6-(2-Phenylethoxy)-2-hexanone*

[2-(4-Bromobutoxy)ethyl]benzene (10.0 g) in dry ether (80 ml) was added dropwise to magnesium turnings (0.946 g) under nitrogen with stirring to give a gentle reflux. The reaction mixture was refluxed for 1 h, allowed to cool to room temperature and added dropwise to acetic anhydride (8.07 g) in dry ether (55 ml) at −70° under nitrogen with stirring over 1.5 h. The reaction mixture was stirred at −70° for 2h, allowed to warm to −10°, then treated with saturated ammonium chloride (100 ml). The organic layer was separated and the aqueous layer re-extracted with ER (150 ml). The combined organic extracts were washed with 2N aqueous sodium hydroxide (150 ml), brine (150 ml), dried and evaporated to give an oil (7.54 g) which was purified by FCC eluting with ER—CX (1:3) to give the *title compound* as a colourless oil (4.34 g). T.l.c (ER—CX 1:3) Rf 0.25.

(ii) *N-[1-Methyl-5-(2-phenylethoxy)pentyl]benzene methanamine*

The product of step (i) (4.16 g) and benzylamine (2.03 g) in toluene (50 ml) was refluxed using a Dean-Stark apparatus for 1 h. The toluene solution in ethanol (100 ml) was hydrogenated over pre-reduced 5% platinum oxide on charcoal (0.40 g). The reaction mixture was filtered (hyflo) and evaporated to give an oil (5.73 g) which was purified by FCC eluting with EA—CX (1:4) + 1% TE to give the *title compound* as a yellow oil (4.51 g). T.l.c. (EA—CX 1:4 + few drops TE) Rf 0.11.

Intermediate 5
2,2,2-Trifluoro-*N*-[6-[2-[4-[4-morpholinyl)phenyl]ethoxy]hexyl]-*N*-(phenylmethyl)-acetamide

A solution of Intermediate 13 (10.0 g), 2-chloroethyl ether (3.38 g), *N,N*-diisopropylethylamine (6.14 g) and sodium iodide (7.11 g) in DMF (500 ml) was stirred at 100° for 2 days under nitrogen. The solvent was evaporated and water (200 ml) was added to the residue. The mixture was extracted with EA (3 × 200 ml) and the combined dried ($Na_2SO_4$) extracts were concentrated to give an oil (16.5 g) which was purified by FCC eluting with ER—CX (1:2) to give the *title compound* as an orange oil (3.49 g). T.l.c. (ER—CX 1:1) Rf 0.26.

Intermediate 6
*N*-[6-[2-[4-(4-Morpholinyl)phenyl]ethoxy]hexyl]benzenemethanamine

Intermediate 5 (3.25 g) in methanol (40 ml) was stirred under nitrogen for 16 h with potassium carbonate (9.0 g). More potassium carbonate (4.5 g) was added and after 24 h water (50 ml) was added. The mixture was extracted with EA (3 × 50 ml) and the combined extracts were washed with water (50 ml) and brine (50 ml), dried ($Na_2SO_4$) and concentrated to give the *title compound* as an orange oil (2.59 g). T.l.c. (EA + few drops TE) Rf 0.18.

Intermediate 7
*N*-[4-[2-[[6-[(Phenylmethyl)amino]hexyl]oxy]ethyl]phenyl]acetamide

Acetic anhydride (1.53 g) in DCM (25 ml) was added dropwise to an ice-cooled solution of Intermediate 13 (6.34 g) in pyridine (1.19 g) and DCM (25 ml) under nitrogen. After 4 h at room temperature the solvent was evaporated and the residual oil in methanol (40 ml) was stirred under nitrogen with potassium carbonate (9.0 g) for 40 h, more potassium carbonate (4.0 g) being added after 24 h. The mixture was diluted with water (100 ml) and extracted with EA (3 × 100 ml). The combined extracts were washed with water (100 ml) and brine (100 ml), dried ($Na_2SO_4$) and concentrated to an oil (5.19 g) which was purified by FCC eluting with (EA—TE 100:1) to give the *title compound* as an orange oil (2.94 g). T.l.c. (FA + few drops TE) Rf 0.15.

Intermediate 8

(a) *N-[(6-(2-Phenylethoxy)hexyl]benzenemethanamine* [2-[(6-Bromohexyl)oxy]ethyl]benzene (4.0 g) was added dropwise to benzylamine (20 ml) at 110°. The solution was heated at 110—120° for 90 min, cooled, and treated with hydrochloric acid (2M; 125 ml). The mixture was extracted with EA (2 × 100 ml) and the extract was washed with aqueous sodium carbonate (100 ml) and brine (100 ml), dried and evaporated. Distillation of the residue gave the *title compound* as a colourless oil (3.2 g) b.p. 180—190°/0.1 mmHg. T.l.c. (CX—ER 1:1) Rf 0.25.

(b) *N-[5-(2-Phenylethoxy)pentyl]benzenemethanamine* (8.6 g) was prepared in a similar manner from [2-[(5-bromopentyl)oxy]ethyl]benzene (10 g) and benzylamine (30 ml). The undistilled product was used without further purification in Example 1(c).

Intermediate 9

(a) *N-[6-[2-(4-Nitrophenyl)ethoxy]hexyl]benzene methanamine*

Intermediate 2(a) (25.9 g) was added dropwise over 40 min to benzylamine (60.76 g) at 120° (bath). After 2 h at 120° the mixture was cooled and water (750 ml) and 2N aqueous hydrochloric acid (375 ml) were added. The mixture was extracted with EA (3 × 800 ml) and the combined extracts were washed with 2N aqueous sodium carbonate (1 l), brine (500 ml), dried ($Na_2SO_4$) and evaporated. The resultant oil (30.4 g) was purified by FCC eluting with EA—CX—TE (25:75:1) to give the *title compound* as an orange oil (22.58 g). T.l.c. (EA—CX 1:2 with a few drops of TE) Rf 0.33.

The following compound was prepared in a similar manner:

(b) *N-[5-[3-[3,5-bis(phenylmethoxy)phenyl]propoxy]pentyl]benzenemethanamine,* by adding Intermediate (2c) (2.5 g) to benzylamine (4.3 ml) at 120° under nitrogen with stirring over 5 min, and keeping the reaction mixture at 120° for 4 h before pouring into the hydrochloric acid-water mixture. Final purification by FCC eluting with EA—CX (1:2) with a few drops of TE gave the *title compound* as a colourless oil (2.44 g). T.l.c. (EA—CX (1:2) + few drops TE) Rf 0.2.

Intermediate 10

(a) *N-[5-[3-(4-Methoxyphenyl)propoxy]pentyl]benzenemethanamine hydrochloride*

1-[3-[(5-Bromopentyl)oxy]propyl]-4-methoxybenzene (2.0 g) was added to benzylamine (6 ml) at 120° under nitrogen. The solution was stirred for 2 h at 120° then added (hot) to 2N hydrochloric acid (50 ml) and water (25 ml). The resulting precipitate was collected by filtration, washed with 2N hydrochloric acid, water and ER then dried at 50° under vacuum to give the *title compound* as a white solid (1.79 g) m.p. 118—121°.

The following compound was prepared in a similar manner:

(b) *N-[5-[2-(4-Methylphenyl)ethoxy]pentyl]benzene methanamine hydrochloride* (8.41 g) as a white solid, m.p. 138°, from Intermediate 2(b) and benzylamine (20 ml).

Intermediate 11

N-[5-(2-(4-Fluorophenyl)ethoxy]pentyl]benzenemethanamine hydrochloride

1-[2-[(5-Bromopentyl)oxy]ethyl]-4-fluorobenzene (7.95 g) was added dropwise over 10 min to benzylamine (24 ml) at 125° under nitrogen. The reaction mixture was stirred for 3.5 h at 120° and the hot reaction mixture was poured into 2N aqueous hydrochloric acid (170 ml) and water (220 ml). After stirring for 15 min the precipitate was collected by filtration to give the *title compound* as a white solid (7.41 g) m.p. 112°.

Intermediate 12

1,1-Dimethyl-5-[2-[4-(4-morpholinyl)phenyl]ethoxy]pentanamine

(i) 4-(4-Morpholinyl)benzeneethanol

4-Aminobenzeneethanol (20.2 g), 2-chloroethyl ether (21.1 g), *N,N*-diisopropylethylamine (38.1 g) and potassium iodide (48.8 g) in DMF were heated to 80° under nitrogen for 60 h. The solvent was evaporated and the residue (~ 143 g) was purified by FCC eluting with ER—CX (1:1 → 1:0) to give the *title compound* as a pink-white solid (17.7 g) m.p. 57°.

(ii) 4-[4-[2-[(4-Bromobutyl)oxy]ethyl]phenyl]morpholine

The product of stage (i) (17.6 g), 1,4-dibromobutane (30 ml), 12.5M aqueous sodium hydroxide (100 ml) and tetra-n-butylammonium bisulphate (2.0 g) were stirred rapidly at room temperature for 16 h. The mixture was diluted with water (400 ml), extracted with ER (3 × 400 ml) and the combined extracts were washed consecutively with water (400 ml) and brine (400 ml), dried and concentrated to give an oil (53.5 g) which was purified by FCC eluting with ER—CX (0.1 → 1:5) to give the *title compound* as an orange oil (20.1 g). T.l.c. ER-hexane (1:5) Rf 0.1.

(iii) 2,2-Dimethyl-6-[2-[4-(4-morpholinyl)phenyl]ethoxy]hexanoic acid

n-Butyllithium in hexane (1.53M, 113.5 ml) was added dropwise to *N,N*-diisopropylamine (17.9 g) in THF (100 ml) at −78° under nitrogen. The mixture was warmed to 0°, stirred for 1 h and treated dropwise with isobutyric acid (7.65 g) in THF (20 ml). The resulting suspension was stirred at room temperature for 3 h, and the product of stage (ii) (20.0 g) was added dropwise. The reaction mixture was stirred for 16 h at room temperature and the solvent was evaporated. The resultant oil was partitioned between EA (250 ml) and water (250 ml). The aqueous layer was acidified to pH 6 with 2N aqueous hydrochloric acid and the organic extract was separated. The aqueous layer was extracted with EA (250 ml) and the combined extracts were concentrated to leave the *title compound* as an orange oil (20.4 g). T.l.c (FR) Rf 0.53.

(iv) (Phenylmethyl) [5-[2-[4-(4-morpholinyl)phenyl]ethoxypentyl]carbamate

Ethyl chloroformate (3.29 g) in acetone (10 ml) was added dropwise to a solution of the product of stage (iii) (10.0 g) and triethylamine (3.0 g, 30 mmol) in acetone (100 ml) and water (10 ml) at 0°. The mixture was stirred at 0° for 40 min and sodium azide (2.0 g) in water (25 ml) was added dropwise. The resulting suspension was stirred at room temperature for 45 min, diluted with water (200 ml) and extracted with T (2 × 200 ml). The dried ($Na_2SO_4$) extract was heated at 75°—80° for 2.5 h and evaporated. The residue was

treated with benzyl alcohol (20 ml), heated at 75°—80° for 60 h and benzyl alcohol was removed by distillation (~ 1 mmHg). The resulting oil was purified on a column of silica (Merck 9385) eluted with ER—CX (1:2) to give the *title compound*, as a yellow oil (4.74 g). T.l.c. ER—CX (1:2) Rf 0.13.

(v) 1,1-Dimethyl-5-[2-[4-(4-morpholinyl)phenyl]ethoxy]pentanamine

The product of stage (iv) (7.50 g) in ethanol (80 ml) was hydrogenated over 10% palladium on charcoal (50% paste in water, 1.0 g). The reaction mixture was filtered (hyflo and the solvent was evaporated to give an oil (5.99 g) which was purified by FCC eluting with EA—ME—TE (66:33:1) to give the *title compound* as a yellow oil (2.84 g). T.l.c. EA—ME—TE (66:33:1) Rf 0.26.

Intermediate 13

N-[6-[2-(4-Aminophenyl)ethoxy]hexyl]-2,2,2-trifluoro-N-phenylmethyl)acetamide

(i) 2,2,2-Trifluoro-*N*-[6-[2-(4-nitrophenyl)ethoxy]hexyl]-*N*-(phenylmethyl)acetamide

Intermediate 2(a) (5.2 g) was added dropwise over 30 min to benzylamine (12.25 g) at 120° (bath). The mixture was maintained at 120° for 2 h, cooled and water (150 ml) and 2N aqueous hydrochloric acid (75 ml) were added. The mixture was extracted with EA (2 × 200 ml, 1 × 100 ml) and the combined extracts were washed with 2N aqueous sodium carbonate (200 ml), brine (200 ml), dried ($Na_2SO_4$) and evaporated to give an oil (5.76 g). The oil in DCM (15 ml) and TE (2.5 ml) was ice-cooled and treated with trifluoroacetic anhydride (2.55 ml) in DCM (10 ml) over 5 min. The reaction mixture was stirred for a further 1 h at room temperature. After 64 h DCM (20 ml) was added and the mixture was washed with 2N aqueous hydrochloric acid (20 ml), 8% aqueous sodium bicarbonate (20 ml), water (20 ml), brine (20 m), dried ($Na_2SO_4$) and evaporated to give an oil (7.46 g), which was purified by FCC eluting with EA—CX—TE (20:80:1) to give *the title compound* as a yellow oil (5.91 g). T.l.c. (EA—CX (1:2) + few drops TE) Rf 0.45.

(ii) N-[6-[2-(4-Aminophenyl)ethoxy]hexyl]-2,2,2-trifluoro-*N*-(phenylmethyl)acetamide

A solution of the product of step (i) (4.95 g) in ethanol (100 ml) was hydrogenated at ambient temperature and pressure over pre-reduced 5% platinum on charcoal (0.5 g). The reaction mixture was filtered (hyflo) and evaporated to give an oil (3.79 g), which was purified by FCC eluting with EA—CX (1:2) with 1% TE to give *the title compound* as a yellow oil (3.57 g). T.l.c. (1:2) + few drops TE) Rf 0.24.

Intermediate 14

N-[5-[2-[4-Methoxyphenyl]ethoxy]pentyl]benzene methanamine hydrochloride

(i) 1-[2-[(5-Bromopentyl)oxy]ethyl]-4-methoxybenzene

A mixture of 4-methoxybenzeneethanol (7.0 g), 1,5-dibromopentane (20 ml), 50% aqueous sodium hydroxide (30 ml) and tetrabutylammonium bisulphate (1 g) was stirred at room temperature overnight, water (100 ml) was added and the mixture was extracted with ER (2 × 100 ml). The organic extracts were washed with water and brine, dried and concentrated *in vacuo* to give an oil which was purified by FCC eluting with hexane → hexane-ER (9:1) to give the *title compound* as a colourless liquid (10.5 g). T.l.c. hexane-ER (9:1) Rf 0.28.

(ii) *N*-[5-[2-[4-Methoxyphenyl]ethoxy]pentylbenzenemethanamine hydrochloride

The product of stage (i) (5 g) was added to benzylamine (15 ml) at 140° under nitrogen. After 2h the reaction mixture was poured into 2N hydrochloric (150 ml) and water (150 ml). The precipitate was collected by filtration, washed with water and ER then dried under vacuum at 50° to give the *title compound* as a white solid (3.4 g) m.p. 123—126°.

Intermediate 15

1-Bromo-6-[(2-propynyl)oxy]hexane, (15.0 g) from propargyl alcohol (5.6 g) and 1,6-dibromohexane (73.2 g) in a similar manner to Intermediate 2a. Purification by FCC eluting with CX followed by CX—ER (19:1). T.l.c. (CX—FR 9:1) Rf 0.4.

Intermediate 16

N-[6-[(2-Propynyl)oxy]hexyl]benzenemethanamine

Intermediate 15 (1.5 g) was added dropwise to benzylamine (10 ml) at 120°. The solution was stirred at *ca* 120° for 1 h, cooled, and added to hydrochloric acid (2M; 50 ml). The mixture was basified with aqueous sodium hydroxide (2M) and extracted with ER (2 × 200 ml). The dried extract was evaporated and excess benzylamine was removed under reduced pressure (*ca* 10 ml). The residue was purified by FCC eluting with ER to give the *title compound* (0.96 g). T.l.c. (ER) Rf 0.1.

Intermediate 17

Ethyl 4-[3-[[6-[(phenylmethylamino]hexyl]oxy]1-butynyl]benzoate

Ethyl 4-iodobenzoate (4.65 g), Intermediate 16 (4.12 g), bis(triphenylphosphino)palladium(II)chloride (120 mg) and copper (I) iodide (70 mg) in diethylamine (90 ml) were stirred under nitrogen at room temperature for 16 h. ER (100 ml) was added and the precipitate was collected by filtration. The filtrate was concentrated and the resultant oil was purified by FCC eluting with ER—TE (100:1) to give the *title*

*compound* as an orange oil (5.96). T.l.c. (ER—TE 100:1). Rf 0.16.

Intermediate 18

Ethyl 4-[3-[[6-[[(4-amino-3,5-dichlorophenyl)2-hydroxyethyl](phenylmethyl)amino]hexyl]oxy]1-propynyl])benzoate

1-(4-amino-3,5-dichlorophenyl)-2-bromoethanone (3.08 g), Intermediate 17 (4.29 g), and *N,N*-diisopropylethylamine (1.41 g) in THF (50 ml) was left to stand for 16 h under nitrogen at room temperature. The precipitate was collected by filtration and the filtrate was concentrated. The resulting oil in ME (60 ml) was ice-cooled and treated portionwise with sodium borohydride (1.55 g), stirred under nitrogen for 24 h and water (200 ml) was added. The mixture was extracted with EA (3 × 150 ml) and, the combined extracts were washed with water (200 ml) and brine (200 ml), dried ($Na_2SO_4$) and concentrated to give an oil (6.48 g) which was purified by FCC eluting with T to give impure *title compound* as a red oil (5.06 g). T.l.c. T Rf 0.2.

Example 1

(a) *4 - Amino - 3,5 - dichloro - α - [[[6 - [2 - (4 - nitrophenyl)ethoxy]hexyl](phenylmethyl)-amino]methyl]benzenemethanol* Intermediate 1 (794 mg), Intermediate 9(a) (1.0 g) and N,N-diisopropylethylamine (400 mg) in THF (15 ml) were left at room temperature overnight. The mixture was filtered and the filtrate concentrated *in vacuo* to an oil which was dissolved in methanol (10 ml), cooled in an ice-bath, treated with sodium borohydride (200 mg) and stirred overnight at room temperature. Water (25 ml) was added and the mixture was extracted with EA (3 × 20 ml). The organic extracts were washed with brine, dried ($Na_2SO_4$) and concentrated to an oil which was purified by FCC eluting with CX—EA—TE (80:20:1) to give the *title compound* as an orange oil (1.3 g). T.l.c. (CX—EA—TE 80:20:1) Rf 0.21. The following compounds were prepared in a similar manner:

(b) *4 - Amino - 3,5 - dichloro - α - [[[6 - (2 - phenylethoxy)hexyl](phenylmethylamino]methyl]-benzenemethanol* (630 mg) as a colourless oil (t.l.c. (CX—EA—TE 80:20:1) Rf 0.23), from Intermediate 1 (500 mg) and Intermediate 8(a) (607 mg), except that the reaction mixture was decanted from the precipitated needles, which were further washed with ether, before concentrating to an oil, and the reaction with sodium borohydride (100 mg) was carried out for only 1 h.

(c) *4 - Amino - 3,5 - dichloro - α - [[[5 - (2 - phenylethoxy)pentyl](phenylmethyl)amino]methyl]-benzenemethanol* (690 mg) as a colourless oil (t.l.c. (CX—EA—TE 66:33:1) Rf 0.25) from Intermediate 1 (500 mg) and Intermediate 8(b) (550 mg), allowing the reaction mixture to stand at room temperature for 5 h, then adding ER (25 ml) before filtering, and evaporating the filtrate. Purification by FCC using CX—EA—TE (66:33:1) as eluent.

(d) *4 - Amino - 3,5 - dichloro - α - [[[6 - (4 - phenylbutoxy)hexyl](phenylmethyl)amino]methyl-benzenemethanol* (1.37 g) as a yellow oil (t.l.c. (EA—CX (1:4) + few drops TE (Rf 0.3) from Intermediate 1 (1.00 g), N-[6-(4-phenylbutoxy)hexyl]benzenemethanamine (1.32 g) and *N,N*-diisopropylethylamine (0.68 ml) in THF (25 ml) leaving the reaction mixture for 72 h at room temperature under nitrogen, before filtering, and evaporating the filtrate. The resultant residue was dissolved in methanol (20 ml) and treated with sodium borohyride (0.267 g), stirred under nitrogen at room temperature for 3 h and more sodium borohydride (0.108 g) was added. After 1 the reaction was worked up by the method of Example 1(a).

(e) *4 - Amino - 3,5 - dichloro - α - [[[1 - methyl - 5 - (2 - phenylethoxy)pentyl](phenylmethyl)aminomethyl]benzenemethanol* (970 mg) as a colourless oil (t.l.c. CX—EA—TE (80:20:1) Rf 0.58), from Intermediate 1 (3.1 g) and Intermediate 4 (3.4 g). Purification by FCC eluting with CX—EA—TE (90:10:1).

(f) *4 - Amino - 3,5 - dichloro - α - [[[5 - [2 - (4 - Fluorophenyl)ethoxy]pentyl](phenylmethyl)amino]-methyl]benzenemethanol* (0.804 g) as a colourless oil (t.l.c. (EA—CX—TE 20:80:1) Rf 0.27, from Intermediate 1 (1.00 g), Intermediate 11 (1.11 g) and *N,N*-diisopropylethylamine (0.912 g), except that the reaction mixture was stirred under nitrogen at room temperature for 16 h, and the reaction with sodium borohydride was allowed to proceed for only 3 h.

(g) *4 - Amino - 3,5 - dichloro - α - [[[6 - [2 - [4 - (4 - morpholinylphenyl]ethoxy]-hexyl](phenylmethyl)amino]methyl]benzenemethanol* (1.75 g) as a viscous oil (t.l.c. H—EA (4:1) Rf (0.11) from Intermediate 1 (1.0 g) and Intermediate 6 (1.5 g). For treatment with sodium borohydride, the residue was dissolved in ME (40 ml) and THF (10 ml) and, following the reaction, the mixture was concentrated to an oil which was partitioned between EA (50 ml) and water (50 m). Final purification by FCC eluting with H—EA (9:1 → 4:1).

(h) *N - [4 - [2 - [[6 - [[2 - (4 - Amino - 3,5 - dichlorophenyl) - 2 - hydroxyethyl](phenylmethyl]-amino]hexyl]oxy]ethyl]phenyl]acetamide* (1.13 g) as a yellow oil (t.l.c. EA—CX (1:1) + few drops TE Rf 0.2), from Intermediate 1 (1.00 g) and Intermediate 7 (1.30 g). The sodium borohydride-methanol reaction mixture was stirred under nitrogen for 24 h. Final purification by FCC eluting with EA—CX—TE (50:50:1).

(i) *4 - Amino - 3,5 - dichloro - α - [[[5 - [2 - (4 - methylphenyl)ethoxy]pentyl(phenylmethyl)amino]-methyl]benzenemethanol* (0.95 g) as a colourless oil (t.l.c. EA—CX—TE (20:80:1) Rf 0.37), from Intermediate 1 (1.00 g), Intermediate 10(b) (1.23 g) and N,N-diisopropylethylamine (0.912 g). The sodium borohydride-methanol reaction mixture was stirred under nitrogen for 48 h. Final purification by FCC eluting with EA—CX (1:6) with 1% TE.

(j) 4 - Amino - α - [[[5 - [3 - [3,5 - bis(phenylmethoxy)phenyl)propoxy]pentyl](phenylmethyl)amino]-methyl] - 3,5 - dichlorobenzenemethanol (2.28 g) as a yellow oil (t.l.c. EA—CX (1:4) + few drops TE Rf

(0.23) from Intermediate 1 (1.0 g) and Intermediate 9(b) (1.85 g). The sodium borohydride-methanol reaction was allowed to proceed for 24 h.

(k) *4 - Amino - 3,5 - dichloro - α - [[[5 - [3 - (4 - methoxyphenyl)propoxy]pentyl](phenylmethyl)-amino]methyl]benzenemethanol* (510 mg) as a colourless oil (t.l.c. CX—EA (9:1) Rf 0.36) from Intermediate 1 (600 mg), Intermediate 10(a) (950 mg) and N,N-diisopropylethylamine (650 mg). After the sodium borohydride-methanol reaction the mixture was concentrated to an oil and partitioned between water (50 ml) and EA (50 ml). Final purification by FCC eluting with CX—EA (9:1).

(l) *4 - Amino - 3,5 - dichloro - α - [[[5 - [2 - (4 - methoxyphenyl)ethoxy]pentyl](phenylmethyl)amino]-methyl]benzenemethanol* (240 mg) as a colourless oil (t.l.c. (hexane-EA—TE (80:20:1) Rf 0.28, from Intermediate 1 (325 mg), Intermediate 14 (430 mg) and *N,N*-diisopropylethylamine (300 mg), except that the reaction with sodium borohydride was carried out for only 6 h, after which the solvent was evaporated and the residue was partitioned between 8% aqueous sodium bicarbonate (25 ml) and ethyl acetate (25 ml). Purification by FCC using hexane-EA—TE (90:10:1) as eluent. T.l.c. silica (hexane-EA—TE 80:20:1) Rf 0.28.

## Example 2

(a) *4-Amino-3,5-dichloro-α-[[[6-(2-phenylethoxy)hexyl]amino]methyl]benzenemethanol*

Example 1(b) (300 mg) was hydrogenated over pre-reduced 10% palladium oxide on carbon (50% aqueous paste 40 mg) in ethanol (20 ml) containing hydrochloric acid (0.6 mmol). After 1.5 h the catalyst was removed by filtration through hyflo and the filtrate was concentrated *in vacuo*. The residue was dissolved in EA (20 ml) and washed with 8% aqueous sodium bicarbonate and brine, dried ($Na_2SO_4$) and evaporated to an oil. Purification by FCC eluting with EA—ME—TE (90:10:1) gave the *title compound* as a white (120 mg) m.p. 60—63°, T.l.c. (EA—ME—TE 80:20:1) Rf 0.42.

(b) *4-amino-3,5-dichloro-α-[[[5-(2-phenylethoxy)pentyl]amino]methyl]benzenemethanol*, a cream solid (370 mg), m.p. 64—65°, t.l.c. (EA—ME—TE 80:20:1) Rf 0.40 was similarly prepared from Example 1(c) (650 mg).

## Example 3

4-Amino-α-[[[6-[2-(4-aminophenyl)ethoxy]hexyl]amino]methyl]-3,5-dichlorobenzenemethanol

Example 1(a) (300 mg) was hydrogenated over pre-reduced 10% palladium oxide on carbon (50% aqueous paste, 60 mg) in ethanol (20 ml) containing hydrochloric acid (1:9 conc. HCl/ethanol, 1 ml). The catalyst was removed by filtration through hyflo and the ethanol was removed under vacuum. 8% Aqueous sodium bicarbonate (20 ml) was added to the residue, which was then extracted with EA (2 × 20 ml). The organic extracts were washed with water and brine, dried ($Na_2SO_4$) and concentrated to a yellow oil which was purified by FCC eluting with EA—ME—TE (80:20:1) to give a solid. Trituration with ER gave the *title compound* as an off-white powder (160 mg) m.p. 71—73°. T.l.c. (EA—ME—TE 80:20:1) Rf 0.32.

## Example 4

(a) *4-Amino-3,5-dichloro-α-[[[1-methyl-5-(2-phenylethoxy)pentyl]amino]methyl]benzenemethanol*

Example 1(e) (890 mg) was hydrogenated over pre-reduced 10% palladium oxide on carbon (50% aqueous paste, 100 mg) in ethanol (20 ml) containing hydrochloride acid (conc. HCl/ethanol, 1:9 v/v, 1.6 ml). The catalyst was removed by filtration through hyflo and the ethanol was evaporated. The residue was partitioned between 8% aqueous sodium bicarbonate and EA. The aqueous layer was re-extracted with EA and the combined organic extracts were washed with brine, dried ($Na_2SO_4$) and concentrated to give a yellow oil which was purified by FCC eluting with EA—TE (99:1) to yield the *title compound* as a colourless oil (580 mg). T.l.c. (EA—TE 99:1) Rf 0.16.

(b) *4-Amino-3,5-dichloro-α-[[[6-(4-phenylbutoxy)hexyl]amino] methyl]benzene methanol* was similarly prepared from Example 1(d) (320 mg). Purification by FCC eluting with EA—ME—TE (80:20:1) followed by trituration with dry ER and drying *in vacuo* gave the *title compound* as a white powder (180 mg) m.p. 74—76°.

| | | | | |
|---|---|---|---|---|
| Found: | C, 63.38; | H, 7.59; | N, 6.06; | Cl, 15.47; |
| $C_{24}H_{34}Cl_2N_2O_2$ requires: | C, 63.57; | H, 7.56; | N, 6.18; | Cl, 15.64%. |

## Example 5

4-Amino-3,5-dichloro-α-[[[1,1-dimethyl-5-[2-[4-(4-morpholinyl)phenyl]ethoxy]pentyl]amino]methyl]benzenemethanol (Z-butenedioate (salt) 1:1

A solution of 4-amino-3,5-dichloro-α-oxobenzeneacetaldehyde (1.9 g) and Intermediate 12 (2.5 g) in benzene (50 ml) was refluxed in a Dean-Stark apparatus for 1 h. The solvent was evaporated, and the residual oil was dissolved in methanol (50 ml) cooled in an ice-bath and treated portionwise with sodium borohydride (1.5 g). The yellow mixture was stirred at room temperature overnight then concentrated to an oil which was partitioned between water (100 ml) and EA (100 ml). The organic layer was washed with water and brine, dried ($Na_2SO_4$) and concentrated to an oil which was purified by FCC eluting with T—ET-ammonia (80:20:1) to give a pale yellow oil (2.5 g). T.l.c. (T—ET-ammonia 80:20:1) Rf 0.53. A sample of the oil (150 mg) in methanol (2 ml) was treated with a solution of maleic acid (150 mg) in methanol (2 ml). The solvent was removed under vacuum and the residue was triturated with ER to give the *title salt* as a white powder (180 m.g.) m.p. 110—115° (dec).

| | | | | |
|---|---|---|---|---|
| Analysis Found: | C, 57.28; | H, 6.71; | N, 6.32; | Cl, 10.83. |
| $C_{27}H_{39}Cl_2N_3O_3.C_4H_{404}.{}^1\!/_2H_2O$ requires: | C, 57,32; | H, 6.83; | N, 6.47; | Cl, 10.92%. |

Example 6

(a) *4-Amino-3,5-dichloro-α-[[[6-[2-[4-(4-morpholinyl)phenyl]ethoxy]hexyl]amino]methyl]benzenemethanol*

Example 1(g) (1.2 g) was hydrogenated over pre-reduced 10% palladium oxide on carbon (50% aqueous paste, 250 mg) in ethanol (25 ml) containing hydrochloric acid (conc. hydrochloric acid/ethanol 1:9 v/v, 2.0 ml). The catalyst was removed by filtration through hyflo, the ethanol was evaporated and the residue was partitioned between EA (50 ml) and 8% aqueous sodium bicarbonate (50 ml). The organic layer was washed with water and brine, dried ($Na_2SO_4$) and concentrated to a solid which was triturated with ER to give the *title compound* as an off-white powder (820 mg) m.p. 102—103°.

| | | | | |
|---|---|---|---|---|
| Found: | C, 61.00; | H, 7.25; | N, 8.07; | Cl, 13.65. |
| $C_{26}H_{37}Cl_2N_3O_3$ requires: | C, 61.17; | H, 7.31; | N, 8.23; | Cl, 13.89%. |

(b) *N-[4-[2-[[6-[[2-(4-Amino-3,5-dichlorophenyl)-2-hydroxyethyl]amino]hexyl]oxy]ethyl]phenyl]acetamide* (0.60 g) as a white solid m.p. 98—100° was similarly prepared from Example 1(h) (1.08 g) using pre-reduced 10% palladium on charcoal (100 mg) as the catalyst.

| | | | | |
|---|---|---|---|---|
| Found: | C, 59.7; | H, 6.9; | N, 8.4; | Cl, 14.6 |
| $C_{24}H_{33}Cl_2N_2O_3$ requires: | C, 59.8; | H, 6.9; | N, 8.7; | Cl 14.7%. |

Example 7

(a) *4-Amino-3,5-dichloro-α-[[[5-[2-[4-(methylphenyl)ethoxy]pentyl]amino]methyl]benzenemethanol*

Example 1(i) (0.92 g) was hydrogenated over pre-reduced 10% palladium on charcoal (100 mg) in ethanol (20 ml) containing hydrochloric acid (conc. hydrochloric acid/ethanol, 1:9 v/v, 1.62 ml) and the catalyst was removed by filtration (hyflo). The filtrate was concentrated and the residue was partitioned between EA (50 ml) and 8% aqueous sodium bicarbonate (2 × 50 ml). The organic layer was washed with brine (50 ml), dried ($Na_2SO_4$) and concentrated to give an oil (0.7 g) which was purified by FCC eluting with EA—TE (100:1) followed by trituration with ether to give the *title compound* as a white solid (0.46 g) m.p. 78°—79°.

| | | | | |
|---|---|---|---|---|
| Found: | C, 62.2; | H, 6.9; | N, 6.5; | Cl, 16.8. |
| $C_{22}H_{30}Cl_2N_2O_2$ requires: | C, 62.1; | H, 7.1; | N, 6.6; | Cl, 16.07;. |

(b) *4 - Amino - 3,5 - dichloro - α - [[[5 - [3 - (4 - methoxyphenyl)propoxy]pentyl]amino]methyl]-benzenemethanol* was similarly prepared from Example 1(k) (450 mg) using pre-reduced 10% palladium oxide on carbon (50% aqueous paste, 100 mg) as the catalyst. Purification by FCC eluting with T—ET—TE (90:10:1) followed by trituration with dry ER gave the *title compound* as a white solid (150 mg) m.p. 63—64°.

| | | | | |
|---|---|---|---|---|
| Found: | C, 60.30; | H, 7.13; | N, 6.03; | Cl, 15.74. |
| $C_{23}H_{32}Cl_2N_2O_3$ requires: | C, 60.66; | H, 7.08; | N, 6.15; | Cl, 15.57%. |

(c) *4 - Amino - 3,5 - dichloro - α - [[[5 - [2 - (4 - fluorophenyl)ethoxy]pentyl]amino]methyl]-benzenemethanol* was similarly prepared from Example 1(f) (0.783 g) using 10% palladium on charcoal (100 mg) as the catalyst. Final trituration with hexane gave the *title compound* as a white solid (0.225 g) m.p. 75—76°.

| | | | | |
|---|---|---|---|---|
| Analysis Found: | C, 59.90; | H, 6.5; | N, 6.5; | Cl, 16.4. |
| $C_{21}H_{27}Cl_2FN_2O_2$ requires: | C, 58.8; | H, 6.3; | N, 6.5; | Cl, 16.5%. |

Example 8

[3-[[5-[[2-(4-Amino-3,5-dichlorophenyl)-2-hydroxyethyl]aminopentyl]oxy]propyl]-1,3-benzenediol

Example 1(j) (2.20 g) was hydrogenated over pre-reduced 10% palladium oxide on charcoal (200 mg) in ethanol (30 ml) containing hydrochloric acid (conc. HCl/ET 1:9 v/v 2.75 ml). The catalyst was removed by filtration (hyflo). The filtrate was concentrated and the residue was partitioned between EA (50 ml) and 8% aqueous sodium bicarbonate (2 × 50 ml). The organic layer was washed with brine (50 ml) dried ($NaSO_4$) and concentrated to give an oil (1.05 g) which was purified by FCC eluting with EA—ME—TE (90:10:1) to give the *title compound* as a white foam (0.369 g). T.l.c. (EA—ME—TE 90:10:1) Rf 0.2.

| | | | | |
|---|---|---|---|---|
| Found: | C, 57.4; | H, 6.7; | N, 5.9; | Cl, 15.1 |
| $C_{22}H_{30}Cl_2N_2O_4$ requires: | C, 57.8; | H, 6.6; | N 6.1; | Cl, 15.5%. |

Example 9

4-Amino-3,5-dichloro-α-[[[5-(2-phenylethoxy)-3-pentynyl]amino]methyl]benzenemethanol

Intermediate 3 (802 mg) in DMF (2 ml) was added to a stirred solution of 4-amino-α-(aminomethy)-3,5-dichlorobenzenemethanol (1.0 g) and N,N-diisopropylethylamine (650 mg) in DMF (20 ml) at 100° under nitrogen. After 2 h the solvent was evaporated and the residue was purified by FCC twice eluting with T—ET—TE (9:5:1) to give a pale yellow oil which was triturated with H to give the *title compound* as a white solid (80 mg) m.p. 62.5—63°C.

| | | | | |
|---|---|---|---|---|
| Found: | C, 61.39; | , 5.93; | N, 6.79; | Cl, 17.37. |
| $C_{21}H_{24}Cl_2N_2O_2$ requires C, 61.92: | C, 61.92; | H, 5.94; | N, 6.88; | Cl, 17.41%. |

Example 10

4-Amino-3,5-dichloro-α-[[[5-[2-(4-methoxyphenyl)ethoxy]pentyl]amino]methyl]benzenemethanol hydrochloride

Example 1(l) (230 mg) was hydrogenated over 10% palladium oxide on carbon (50% aqueous paste, 40 mg) in ethanol (10 ml). The catalyst was removed by filtration through hyflo and the ethanol was evaporated to give a green solid which was triturated with dry ether to give the *title compound* as a pale green powder (170 mg) m.p. 134—137° (dec).

| | | | | |
|---|---|---|---|---|
| Analysis Found: | C, 54.28; | H, 6.45; | N, 5.58; | Cl, 21.35. |
| $C_{22}H_{30}Cl_2N_2O_3.HCl.\frac{1}{2}H_2O$ requires: | C, 54.27; | H, 6.62; | N 5.75; | Cl, 21.85%. |

Example 11

Ethyl 4-[3-[[6-[[(4-amino-3,5-dichlorophenyl)2-hydroxyethyl]amino]-hexyl]oxy]propyl]benzoate

Intermediate 18 (500 mg) in ET (20 ml) containing hydrochloric acid (conc HCl 1:9 v/v, 0.76 ml) was hydrogenated over pre-reduced 10% palladium on charcoal (50% paste in water, 60 mg). The reaction mixture was filtered (hyflo) and the filtrate was concentrated. The residue was partitioned between EA (50 ml) and 8% aqueous sodium bicarbonate (2 × 50 ml). The organic layer washed with brine (50 ml), dried ($Na_2SO_4$) and concentrated. The residue was purified by FCC eluting with EA—H—TE (50:50:1) to give the *title compound* as a white solid (97 mg) m.p. 66—68°. T.l.c. (EA—H—TE 50:50:1) Rf 0.05.

Example 12

4-Amino-3,5-dichloro-α-[[[6-[3-[4-(hydroxymethyl)phenyl]propoxy]hexyl]amino]methyl]benzenemethanol

The compound of Example 11 (88 mg) in ER (4 ml) was added dropwise to a stirred suspension of lithium aluminium hydride (50 mg) in ER (4 ml) under nitrogen. The reaction mixture was stirred for 2.5 h at room temperature treated dropwise with water (0.05 ml), 2N aqueous sodium sodium hydroxide (0.1 ml) and water (0.1 ml). The mixture was filtered (hyflo) and the filtrate was concentrated to give a colourless oil (64 mg), which on trituration with ER afforded the *title compound* as a white solid m.p. 56—59°. T.l.c. (T—ET—$NH_3$ 39:11:1) Rf 0.44.

The following are examples of suitable formulations of compounds of the invention. The term "active ingredient" is used herein to represent a compound of the invention.

Tablets (Direct Compression)

| | mg/tablet |
|---|---|
| Active ingredient | 2.0 |
| Microcrystalline Cellulose USP | 196.5 |
| Magnesium Stearate BP | 1.5 |
| Compression weight | 200.0 |

The active ingredient is sieved through a suitable sieve, blended with the excipients and compressed using 7 mm diameter punches.

Tablets of other strengths may be prepared by altering the ratio of active ingredient to microcrystalline cellulose or the compression weight and using punches to suit.

The tablets may be film coated with suitable film forming materials, such as hydroxypropylmethylcellulose, using standard techniques. Alternatively the tablets may be sugar coated.

Syrup (Sucrose-free)

| | mg/5ml dose |
|---|---|
| Active ingredient | 2.0 mg |
| Hydroxypropyl methylcellulose USP (viscosity type 4000) | 22.5 mg |

| | mg/5ml dose |
|---|---|
| Buffer<br>Flavour<br>Colour<br>Preservative<br>Sweetener | as required |
| Purified Water BP to | 5.0 ml |

The hydroxypropyl methylcellulose is dispersed in hot water, cooled and then mixed with an aqueous solution containing the active ingredient and the other components of the formulation. The resultant solution is adjusted to volume and mixed. The syrup is clarified by filtration.

Metered Dose Pressurised Aerosol
A. Suspension Aerosol

| | mg/metered dose | Per can |
|---|---|---|
| Active ingredient micronised | 0.100 | 26.40 mg |
| Oleic Acid BP | 0.100 | 2.64 mg |
| Trichlorofluoromethane BP | 23.64 | 5.67 g |
| Dichlorodifluoromethane BP | 61.25 | 14.70 g |

The active ingredient is micronised in a fluid energy mill to a fine particule size range. The Oleic Acid is mixed with the Trichlorofluoromethane at a temperature of 10—15°C and the micronised drug is mixed into the solution with a high shear mixer. The suspension is metered into aluminium aerosol cans and suitable metering valves delivering 85 mg of suspension are crimped onto the cans and the Dichlorodifluoromethane is pressure filled into the cans through the valves.

B. Solution Aerosol

| | mg/metered dose | Per can |
|---|---|---|
| Active ingredient | 0.055 | 13.20 mg |
| Ethanol BP | 11.100 | 2.66 g |
| Dichlorotetrafluoroethane BP | 25.160 | 6.04 g |
| Dichlorodifluoromethane BP | 37.740 | 9.06 g |

Oleic acid BP, or a suitable surfactant e.g. Span 85 (sorbitan trioleate) may also be included.

The active ingredient is dissolved in the ethanol together with the oleic acid or surfactant if used. The alcoholic solution is metered into suitable aerosol containers followed by the dichlorotetrafluoroethane. Suitable metering valves are crimped onto the containers and dichlorodifluoromethane is pressure filled into them through the valves.

Injection for Intravenous Administration

| | mg/ml |
|---|---|
| Active ingredient | 0.5 mg |
| Sodium Chloride BP | as required |
| Water for Injection BP to | 1.0 ml |

Sodium chloride may be added to adjust the tonicity of the solution and the pH may be adjusted, using acid or alkali, to that of optimum stability and/or facilitate solution of the active ingredient. Alternatively suitable buffer salts may be used.

The solution is prepared, clarified and filled into appropriate size ampoules scaled by fusion of the glass. The injection is sterilised by heat in an autoclave using one of the acceptable cycles. Alternatively the solution may be sterilised by filtration and filled into sterile ampoules under asoptic conditions. The solution may be packed under an inert atmosphere of nitrogen or other suitable gas.

Inhalation Cartridges

| | mg/cartridge |
|---|---|
| Active ingredient micronised | 0.200 |
| Lactose BP to | 25.0 |

The active ingredient is micronised in a fluid energy mill to a fine particle size range prior to blending with normal tabletting grade lactose in a high energy mixer. The powder blend is filled into No. 3 hard gelatin capsules on a suitable encapsulating machine. The contents of the cartridges are administered using a powder inhaler such as the Glaxo Rotahaler.

**Claims for the Contracting States: BE CH DE FR IT LI LU NL SE**

1. Compounds of the general formula (I)

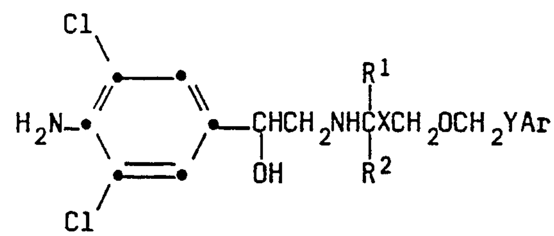

(I)

wherein

X represents a $C_{1-6}$ alkylene, $C_{2-6}$ alkenylene or $C_{2-6}$ alkynylene chain and

Y represents a bond, or a $C_{1-4}$ alkylene, $C_{2-4}$ alkenylene or $C_{2-4}$ alkynylene chain with the proviso that the sum total of carbon atoms in X and Y is not more than 8;

Ar represents a phenyl group optionally substituted by one or more substituents selected from halogen atoms, or the groups $C_{1-3}$ alkyl, $C_{1-3}$ alkoxy, $—(CH_2)_qOH$ (where q represents an integer from 0 to 3), $—NR^3R^4$ (where $R^3$ and $R^4$ each represents a hydrogen atom, or a $C_{1-4}$ alkyl group, or $NR^3R^4$ forms a saturated heterocyclic amino group which has 5-7 ring members and optionally contains in the ring one or more atoms selected from —O— or —S— or a group —NH— or $—N(CH_3)—$, or $—NR^5COR^6$ (where $R^5$ represents a hydrogen atom or a $C_{1-4}$ alkyl group, and $R^6$ represents a hydrogen atom or a $C_{1-4}$ alkyl group, and $R^6$ represents a hydrogen atom or a $C_{1-4}$ alkyl, $C_{1-4}$ alkoxy, phenyl or $—NR^3R^4$ group), or Ar is a phenyl group substituted by an alkylenedioxy group of formula $—O(CH_2)_pO—$, where p is 1 or 2;

$R^1$ and $R^2$ each represents a hydrogen atom or a $C_{1-3}$ alkyl group, with the proviso that the sum total of carbon atoms in $R^1$ and $R^2$ is not more than 4;

and physiologically acceptable salts and solvates thereof.

2. Compounds as claimed in claim 1, in which the chain X is $—(CH_2)_2—$, $—(CH_2)_3—$, $—(CH_2)_4—$, $—(CH_2)_5—$, $—(CH_2)_6—$, $—CH_2C{\equiv}C—$, $—(CH_2)_2CH{=}CH—$, $—(CH_2)_2C{\equiv}C—$, $—CH{=}CHCH_2—$, $—CH{=}CH(CH_2)_2—$ or $—CH_2C{\equiv}CCH_2—$ and the chain Y is $—CH_2—$, $—(CH_2)_2—$, $—(CH_2)_3—$, $—(CH_2)_4—$, $—CH{=}CH—$, $—C{\equiv}C—$, $—CH_2CH{=}CH—$, or $—CH_2C{\equiv}C—$.

3. Compounds as claimed in claim 1, in which X is a $C_{2-6}$ alkylene or $C_{2-6}$ alkynylene chain and Y is a $C_{1-4}$ alkylene chain.

4. Compounds as claimed in any of claims 1 to 3, in which $R^1$ and $R^2$ independently represent a hydrogen atom or a methyl group.

5. Compounds as claimed in any of claims 1 to 4, in which Ar is an unsubstituted phenyl group or is a phenyl group having one, two or three substituents selected from bromine, iodine, chlorine, fluorine, methyl, ethyl, methoxy, ethoxy, amino, methylamino, ethylamino, dimethylamino, diethylamino, morpholino, piperidino, piperazino, N-methylpiperazino, $—NHCOR^6$ (where $R^6$ is hydrogen, $C_{1-4}$ alkyl, $C_{1-4}$ alkoxy, phenyl or amino), hydroxy, $—CH_2OH$, or $—(CH_2)_2OH$.

6. Compounds of the general formula (1a)

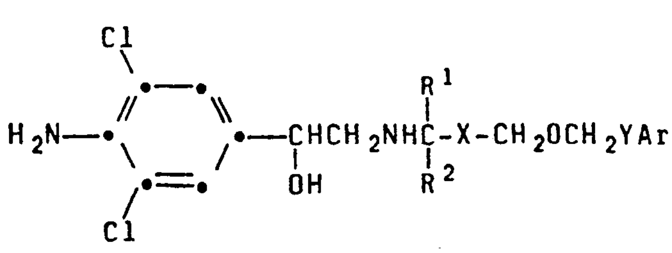

(Ia)

wherein

X represents a $C_{3-4}$ alkylene or $C_3$ alkynylene chain;

Y represents a $C_{1-3}$ alkylene chain;

$R^1$ and $R^2$ represent hydrogen or methyl; and

Ar represents a phenyl group optionally substituted by a flourine atom, a group selected from amino, $C_{1-3}$ alkyl, $C_{1-3}$ alkoxy, hydroxy$C_{1-2}$alkyl, morpholino, hydroxy or —NHCOR$^6$ where $R^6$ is $C_{1-3}$ alkyl, or Ar is a phenyl group substituted by hydroxyl groups at the 3- and 5- positions; and physiologically acceptable salts and solvates thereof.

7. Compounds as claimed in claim 6 in which Ar is a phenyl group optionally containing one substituent at the 4- position, which is an amino, —NHAcetyl or morpholino group.

8. The compounds:

4-amino-3,5-dichloro-α-[[[6-[2-[4-(4-morpholinyl)phenyl]ethoxy]hexyl]amino]methyl]benzenemethanol;

N-[4-[2-[[6-[[2-(4-amino-3,5-dichlorophenyl)-2-hydroxyethyl]amino]hexyl]oxy]ethyl]phenyl]acetamide;

4-amino-3,5-dichloro-α-[[[6-[2-(4-aminophenyl)ethoxy]hexyl]amino]methyl]benzenemethanol;

4-amino-3,5-dichloro-α-[[[5-(2-phenylethoxy]pentyl]amino]methyl]benzenemethanol;

4-amino-3,5-dichloro-α-[[[6-(2-phenylethoxy)hexyl]amino]methyl]benzenemethanol;

4-amino-3,5-dichloro-α-[[[1-methyl-5-(2-phenylethoxy)pentyl]amino]methyl]benzenemethanol;

4-amino-3,5-dichloro-α-[[[5-(2-phenylethoxy)-3-pentynyl]amino]methyl]benzenemethanol;

[3-[[5-[[2-(4-amino-3,5-dichlorophenyl)-2-hydroxyethyl]amino]pentyl]oxy]propyl]-1,3-benzenediol;

and physiologically acceptable salts and solvates thereof.

9. A process for the preparation of compounds as claimed in any of claims 1 to 8 or a physiologically acceptable salt or solvate thereof which comprises:

(1a) for the preparation of compounds of formula (I) in which $R^1$ is a hydrogen atom, alkylating an amine of general formula (II)

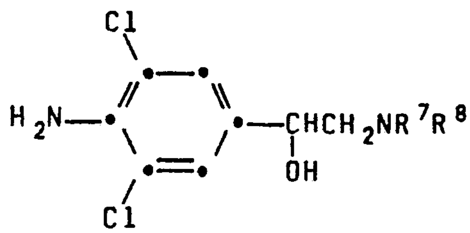

(II)

(wherein $R^7$ is a hydrogen atom or a protecting group and $R^8$ is a hydrogen atom) with an alkylating agent of general formula (III).

$$\underset{\displaystyle R^2}{\underset{|}{LCH}}XCH_2OCH_2YAr \qquad (III)$$

(where L is a leaving group) followed, if necessary by removal of any protecting groups present; or

(1b) for the preparation of compounds of formula (I) in which $R^1$ is a hydrogen atom, alkylating an amine of general formula (II) in which $R^7$ is a hydrogen atom or a protecting group and $R^8$ is a hydrogen atom or a group convertible thereto under the reaction conditions, with a compound of general formula (IV)

$$R^2COXCH_2OCH_2YAr \qquad (IV)$$

in the presence of a reducing agent followed, if necessary, by removal of any protecting groups present; or

(2) reducing an intermediate of general formula (VI)

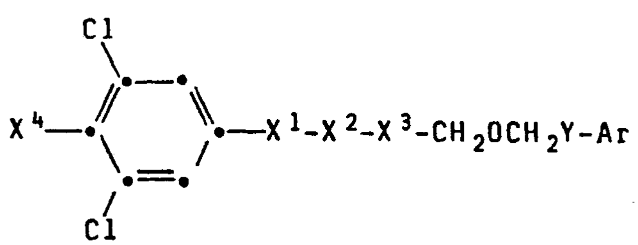

(VI)

wherein

$X^1$ is —CH(OH)— or a group convertible thereto by reduction,

$X^2$ is —$CH_2NR^7$— or a group convertible thereto by reduction,

$X^3$ is $—CR^1R^2X—$ or a group convertible thereto by reduction,

$X^4$ is $—NH_2$ or a group convertible thereto by reduction,

Y is as defined in claim 1 or is a group convertible thereto by reduction and

Ar is as defined in claim 1 or is a group convertible thereto by reduction,

at least one of $X^1$, $X^2$, $X^3$, $X^4$, Y and Ar containing a reducible group, followed, if necessary, by removal of any protecting groups present; and, if desired, converting the resulting compound of general formula (I) or a salt thereof into a physiologically acceptable salt or solvate thereof.

10. A pharmaceutical composition comprising at least one compound of general formula (I) as defined in any of claims 1 to 8 or a physiologically acceptable salt or solvate thereof, together with a physiologically acceptable carrier or excipient.

**Claims for the Contracting State: AT**

1. A process for the preparation of compounds of the general formula (I)

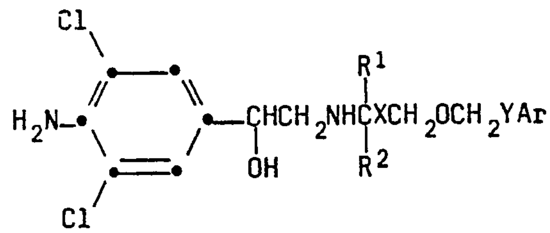

(I)

wherein

X represents a $C_{1-6}$ alkylene, $C_{2-6}$ alkenylene or $C_{2-6}$ alkynylene chain and

Y represents a bond, or a $C_{1-4}$ alkylene, $C_{2-4}$ alkenylene or $C_{2-4}$ alkynylene chain with the proviso that the sum total of carbon atoms in X and Y is not more than 8;

Ar represents a phenyl group optionally substituted by one or more substituents selected from halogen atoms, or the groups $C_{1-3}$ alkyl, $C_{1-3}$ alkoxy, $—(CH_2)_qOH$ (where q represents an integer from 0 to 3), $—NR^3R^4$ (where $R^3$ and $R^4$ each represents a hydrogen atom, or a $C_{1-4}$ alkyl group, or $NR^3R^4$ forms a saturated heterocyclic amino group which has 5-7 ring members and optionally contains in the ring one or more atoms selected from —O— or —S— or a group —NH— or $—N(CH_3)—$), or $—NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $—NR^3R^4$ group), or Ar is a phenyl group substituted by an alkylenedioxy group of formula $—O(CH_2)_pO—$, where p is 1 or 2;

$R^1$ and $R^2$ each represents a hydrogen atom or a $C_{1-3}$ alkyl group, with the proviso that the sum total of carbon atoms in $R^1$ and $R^2$ is not more than 4;

and physiologically acceptable salts and solvates thereof, which comprises:

(1a) for the preparation of compounds of formula (I) in which $R^1$ is a hydrogen atom, alkylating an amine of general formula (II)

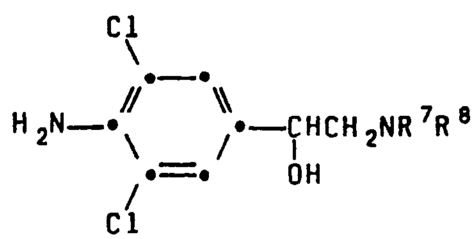

(II)

(wherein $R^7$ is a hydrogen atom or a protecting group and $R^8$ is a hydrogen atom) with an alkylating agent of general formula (III).

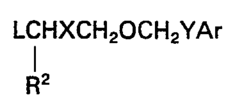

(III)

(where L is a leaving group) followed, if necessary by removal of any protecting groups present; or

(1b) for the preparation of compounds of formula (I) in which $R^1$ is a hydrogen atom, alkylating an amine of general formula (II) in which $R^7$ is a hydrogen atom or a protecting group and $R^8$ is a hydrogen atom or a group convertible thereto under the reaction conditions, with a compound of general formula (IV)

$$R^2COXCH_2OCH_2YAr \qquad (IV)$$

in the presence of a reducing agent followed, if necessary, by removal of any protecting groups present; or

(2) reducing an intermediate of general formula (VI)

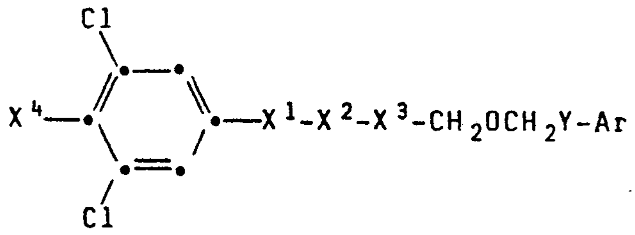

(VI)

wherein

$X^1$ is —CH(OH)— or a group convertible thereto by reduction,

$X^2$ is $—CH_2NR^7—$ or a group convertible thereto by reduction,

$X^3$ is $—CR^1R^2X—$ or a group convertible thereto by reduction,

$X^4$ is $—NH_2$ or a group convertible thereto by reduction,

Y is as defined in claim 1 or is a group convertible thereto by reduction and

Ar is as defined in claim 1 or is a group convertible thereto by reduction,

at least one of $X^1$, $X^2$, $X^3$, $X^4$, Y and Ar containing a reducible group, followed, if necessary, by removal of any protecting groups present; and, if desired, converting the resulting compound of general formula (I) or a salt thereof into a physiologically acceptable salt or solvate thereof.

2. A process as claimed in claim 1, for the preparation of compounds in which the chain X is $—(CH_2)_2—$, $—(CH_2)_3—$, $—(CH_2)_4—$, $—(CH_2)_5—$, $—(CH_2)_6—$, $—CH_2C{\equiv}C—$, $—(CH_2)_2CH{=}CH—$, $—(CH_2)_2C{\equiv}C—$, $—CH{=}CHCH_2—$, $—CH{=}CH(CH_2)_2—$ or $—CH_2C{\equiv}CCH_2—$ and the chain Y is $—CH_2—$, $—(CH_2)_2—$, $—(CH_2)_3—$, $—(CH_2)_4—$, $—CH{=}CH—$, $—C{\equiv}C—$, $—CH_2CH{=}CH—$, or $—CH_2C{\equiv}C—$.

3. A process as claimed in claim 1, for the preparation of compounds in which X is a $C_{2-6}$ alkylene or $C_{2-6}$ alkynylene chain and Y is a $C_{1-4}$ alkylene chain.

4. A process as claimed in any of claims 1 to 3, for the production of compounds in which $R^1$ and $R^2$ independently represent a hydrogen atom or a methyl group.

5. A process as claimed in any one of claims 1 to 3 for the preparation of compounds, in which $R^1$ and $R^2$ are both hydrogen atoms or $R^1$ is a hydrogen atom and $R^2$ is a $C_{1-3}$ alkyl group.

6. A process as claimed in any of claims 1 to 5, for the preparation of compounds in which Ar is an unsubstituted phenyl group or is a phenyl group having one, two or three substituents selected from bromine, iodine, chlorine, fluorine, methyl, ethyl, methoxy, ethoxy, amino, methylamino, ethylamino, dimethylamino, diethylamino, morpholino, piperidino, piperazino, N-methylpiperazino, $—NHCOR^6$ (where $R^6$ is hydrogen, $C_{1-4}$ alkyl, $C_{1-4}$ alkoxy, phenyl or amino), hydroxyl, $—CH_2OH$, or $—(CH_2)_2OH$.

7. A process as claimed in claim 1 for the preparation of compounds of the general formula (1a)

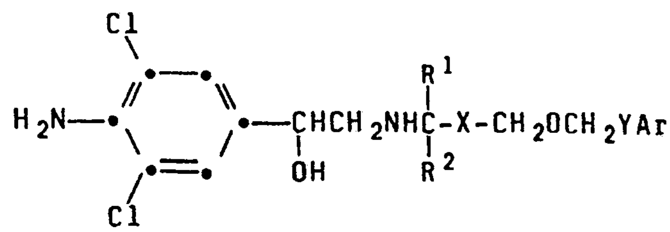

(Ia)

wherein

X represents a $C_{3-4}$ alkylene or $C_3$ alkynylene chain;

Y represents a $C_{1-3}$ alkylene chain;

$R^1$ and $R^2$ represent hydrogen or methyl; and

Ar represents a phenyl group optionally substituted by a flourine atom, a group selected from amino, $C_{1-3}$ alkyl, $C_{1-3}$ alkoxy, hydroxy$C_{1-2}$alkyl, morpholino, hydroxy or $—NHCOR^6$ where $R^6$ is $C_{1-3}$ alkyl, or Ar is a phenyl group substituted by hydroxyl groups at the 3- and 5- positions; and physiologically acceptable salts and solvates thereof.

8. A process as claimed in claim 7 for the preparation of compounds in which Ar is a phenyl group optionally containing one substituent at the 4- position, which is an amino, —NHAcetyl or morpholino group.

9. A process as claimed in claim 1 for the preparation of the compounds:

4-amino-3,5-dichloro-α-[[[6-[2-[4-(4-morpholinyl)phenyl]ethoxy]hexyl]amino]methyl]benzenemethanol;

N-[4-[2-[[6-[[2-(4-amino-3,5-dichlorophenyl)-2-hydroxyethyl]amino]hexyl]oxy]ethyl]phenyl]acetamide;

4-amino-3,5-dichloro-α-[[[6-[2-(4-aminophenyl)ethoxy]hexyl]amino]methyl]benzenemethanol;

4-amino-3,5-dichloro-α-[[[5-(2-phenylethoxy]pentyl]amino]methyl]benzenemethanol;
4-amino-3,5-dichloro-α-[[[6-(2-phenylethoxy)hexyl]amino]methyl]benzenemethanol;
4-amino-3,5-dichloro-α-[[[1-methyl-5-(2-phenylethoxy)pentyl]amino]methyl]benzenemethanol;
4-amino-3,5-dichloro-α-[[[5-(2-phenylethoxy)-3-pentynyl]amino]methyl]benzenemethanol;
[3-[[5-[[2-(4-amino-3,5-dichlorophenyl)-2-hydroxyethyl]amino]pentyl]oxy]propyl]-1,3-benzenediol;
and physiologically acceptable salts and solvates thereof.

**Patentansprüche für die Vertragsstaaten: BE CH DE FR IT LI LU NL SE**

1. Verbindungen der allgemeinen Formel (I)

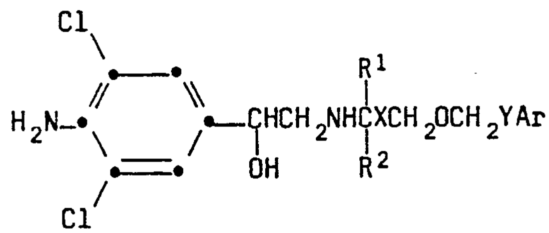

(I)

worin:

X für eine $C_{1-6}$-Alkylen-, $C_{2-6}$-Alkenylen- oder $C_{2-6}$-Alkinylenkette steht und

Y eine Bindung oder eine $C_{1-4}$-Alkylen, $C_{2-4}$-Alkenylen- oder $C_{2-4}$-Alkinylenkette bedeutet, mit der maßgabe, daß die Gesamtsumme der Kohlenstoffatome in X und Y nicht mehr als 8 ist;

Ar für eine Phenylgruppe steht, die gegebenenfalls durch ein oder mehrere Substituenten, ausgewählt aus Halogenatomen oder den Gruppen $C_{1-3}$-Alkyl, $C_{1-3}$-Alkoxy, $—(CH_2)_qOH$ (worin q eine ganze Zahl von 0 bis 3 bedeutet), $—NR^3R^4$ (worin $R^3$ und $R^4$ jeweils ein Wasserstoffatom oder eine $C_{1-4}$-Alkylgruppe bedeuten oder $NR^3R^4$ eine gesättigte heterocyclische Aminogruppe bildet, die 5 bis 7 Ringglieder hat und gegebenenfalls im Ring ein oder mehrere Atome, ausgewählt aus —O— oder —S—, oder eine Gruppe —NH— oder $—N(CH_3)—$ enthält) oder $—NR^5COR^6$ (worin $R^5$ ein Wasserstoffatom oder eine $C_{1-4}$-Alkylgruppe ist und $R^6$ ein Wasserstoffatom oder eine $C_{1-4}$-Alkyl-, $C_{1-4}$-Alkoxy-, Phenyl- oder $—NR^3R^4$-Gruppe ist), substituiert ist, oder Ar eine Phenylgruppe ist, die durch eine Alkylendioxygruppe der Formel $—O(CH_2)_pO—$ substituiert ist, wobei p den Wert 1 oder 2 hat;

$R^1$ und $R^2$ jeweils für ein Wasserstoffatom oder eine $C_{1-3}$-Alkylgruppe mit der Maßgabe stehen, daß die Gesamtsumme der Kohlenstoffatome in $R^1$ und $R^2$ nicht mehr als 4 ist,
und die physiologisch annehmbaren Salze und Solvate davon.

2. Verbindungen nach Anspruch 1, dadurch gekennzeichnet, daß die durch X angegebene Kette $—(CH_2)_2—$, $—(CH_2)_3—$, $—(CH_2)_4—$, $—(CH_2)_5—$, $—(CH_2)_6—$, $—CH_2C{\equiv}C—$, $—(CH_2)_2CH{=}CH$, $—(CH_2)_2C{\equiv}C—$, $—CH{=}CHCH_2—$, $—CH{=}CH(CH_2)_2—$ oder $—CH_2C{\equiv}CCH_2—$ und die durch Y angegebene Kette $—CH_2—$, $—(CH_2)_2—$, $—(CH_2)_3—$, $—(CH_2)_4—$, $—CH{=}CH—$, $—C{\equiv}C—$, $—CH_2CH{=}CH$ oder $—CH_2C{\equiv}C—$ ist.

3. Verbindungen nach Anspruch 1, dadurch gekennzeichnet, daß X eine $C_{2-6}$-Alkylen- oder $C_{2-6}$-Alkinylenkette ist und Y eine $C_{1-4}$-Alkylenkette ist.

4. Verbindung nach einem der Ansprüche 1 bis 3, dadurch gekennzeichnet, daß $R^1$ und $R^2$ unabhängig voneinander für ein Wasserstoffatom oder eine Methylgruppe stehen.

5. Verbindungen nach einem der Ansprüche 1 bis 4, dadurch gekennzeichnet, daß Ar eine unsubstituierte Phenylgruppe ist oder eine Phenylgruppe ist, die ein, zwei oder drei Substituenten, ausgewählt aus Brom, Iod, Chlor, Fluor, Methyl, Ethyl, Methoxy, Ethoxy, Amino, Methylamino, Ethylamino, Dimethylamino, Diethylamino, Morpholino, Piperidino, Piperazino, N-Methylpiperazino, $—NHCOR^6$ (worin $R^6$ für Wasserstoff, $C_{1-4}$-Alkyl, $C_{1-4}$-Alkoxy, Phenyl oder Amino steht), Hydroxy, $—CH_2OH$ oder $—(CH_2)_2OH$, aufweist.

6. Verbindungen der allgemeinen Formel (Ia)

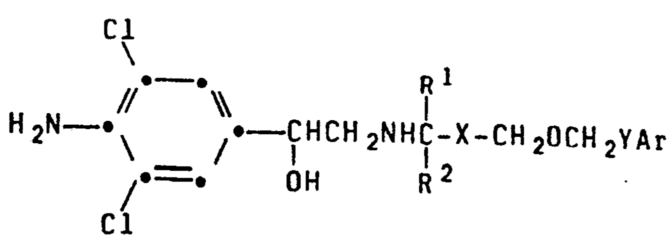

(Ia)

worin

X für eine $C_{3-4}$-Alkylen- oder $C_3$-Alkinylenkette steht;

Y für eine $C_{1-3}$-Alkylenkette steht;

$R^1$ und $R^2$ jeweils für Wasserstoff oder Methyl stehen; und

Ar eine Phenylgruppe bedeutet, die gegebenenfalls durch ein Fluoratom, eine Gruppe, ausgewählt aus Amino, $C_{1-3}$-Alkyl, $C_{1-3}$-Alkoxy, Hydroxy-$C_{1-2}$-alkyl, Morpholino, Hydroxy oder —$NHCOR^6$, wobei $R^6$ für $C_{1-3}$-Alkyl steht, substituiert ist, oder Ar eine Phenylgruppe ist, die durch Hydroxylgruppen in 3- und 5-Positionen substituiert ist,

und die physiologisch annehmbaren Salze und Solvate davon.,

7. Verbindungen nach Anspruch 6, dadurch gekennzeichnet, daß R eine Phenylgruppe ist, die gegebenenfalls einen Substituenten in 4-Position, der eine Amino-, —NH-Acetyl- oder Morpholinogruppe ist, enthält.

8. Die Verbindungen:

4-Amino-3,5-dichlor-α-[[[6-[2-[4-(4-morpholinyl)-phenyl]-ethoxy]-hexyl]-amino]-methyl]-benzolmethanol;

N-[4-[2-[[6-[[2-(4-Amino-3,5-dichlorophenyl)-2-hydroxyethyl]-amino]-hexyl]-oxy]-ethyl]-phenyl]-acetamid;

4-Amino-3,5-dichlor-α-[[[6-[2-(4-aminophenyl)-ethoxy]-hexyl]-amino]-methyl]-benzolmethanol;

4-Amino-3,5-dichlor-α-[[[5-(2-phenylethoxy)-pentyl]-amino]-methyl]-benzolmethanol;

4-Amino-3,5-dichlor-α-[[[6-(2-phenylethoxy)-hexyl]-amino]-methyl]-benzolmethanol;

4-Amino-3,5-dichlor-α-[[[1-methyl-5-(2-phenylethoxy)-pentyl]-amino]-methyl]-benzolmethanol;

4-Amino-3,5-dichlor-α-[[[5-(2-phenylethoxy)-3-pentinyl]-amino]-methyl]-benzolmethanol;

[3-[[5-[[2-(4-Amino-3,5-dichlorphenyl)-2-hydroxyethyl]-amino]-pentyl]-oxy]-propyl]-1,3-benzoldiol

und die physiogisch annehmbaren Salze und Solvate davon.

9. Verfahren zur Herstellung der Verbindungen nach den Ansprüchen 1 bis 8 oder eines physiologisch annehmbaren Salzes oder Solvats davon, dadurch gekennzeichnet, daß man:

(1a) zur Herstellung von Verbindungen der Formel (I), bei denen $R^1$ ein Wasserstoffatom ist, ein Amin der allgemeinen Formel (II)

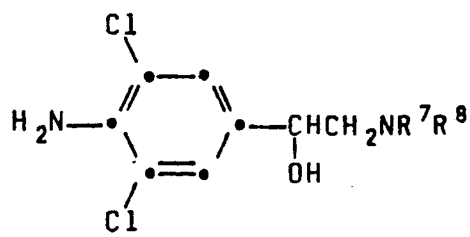

(II)

(worin $R^7$ ein Wasserstoffatom oder eine Schutzgruppe ist und $R^8$ für ein Wasserstoffatom steht) mit einem Alkylierungsmittel der allgemeinen Formel (III)

$$\underset{\displaystyle R^2}{LCH}XCH_2OCH_2YAr \qquad (III)$$

(worin L eine Austrittsgruppe ist) alkyliert und anschließend erforderlichenfalls irgendwelche vorhandenen Schutzgruppen entfernt oder daß man

(1b) zur Herstellung von Verbindungen der Formel (I), bei denen $R^1$ ein Wasserstoffatom ist, ein Amin der allgemeinen Formel (II), bei dem $R^7$ ein Wasserstoffatom oder eine Schutzgruppe ist und $R^8$ ein Wasserstoffatom oder eine in dieses unter Reaktionsbedingungen umwandelbare Gruppe ist, mit einer Verbindung der allgemeinen Formel (IV)

$$R^2COXCH_2OCH_2YAr \qquad (IV)$$

in Gegenwart eines Reduktionsmittels alkyliert und erforderlichenfalls irgendwelche vorhandenen Schutzgruppen entfernt oder daß man

(2) ein Zwischenprodukt der allgemeinen Formel (VI)

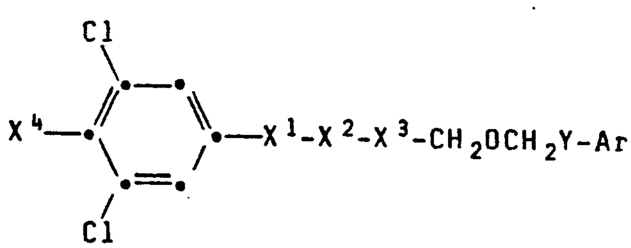

(VI)

worin

$X^1$ für —CH(OH)— oder eine in diese Gruppe durch Reduktion umwandelbare Gruppe steht,

$X^2$ für $CH_2NR^7$— oder eine in diese Gruppe durch Reduktion umwandelbare Gruppe steht,

$X^3$ für —$CR^1R^2X$— oder eine in diese Gruppe durch Reduktion umwandelbare Gruppe steht,

$X^4$ für —$NH_2$ oder eine in diese Gruppe durch Reduktion umwandelbare Gruppe steht, Y wie in Anspruch 1 definiert ist oder eine Gruppe ist, die durch Reduktion in eine solche Gruppe umwandelbar ist, und Ar wie in Anspruch 1 definiert ist oder eine in eine solche Gruppe durch Reduktion umwandelbare Gruppe ist,

mindestens eine von $X^1$, $X^2$, $X^3$, $X^4$, Y und Ar eine reduzierbare Gurppe enthält, reduziert und anschließend erforderlichenfalls irgendwelche vorhandenen Schutzgruppen entfernt und daß man

gewünschtenfalls die resultierende Verbindung der allgemeinen Formel (I) oder ein Salz davon in ein physiologisch annehmbares Salz oder Solvat davon umwandelt.

10. Pharmazeutisches Präparat, dadurch gekennzeichnet, daß es mindestens ein Verbindung der allgemeinen Formel (I) nach einem der Ansprüche 1 bis 8 oder ein physiologisch annehmbares Salz oder Solvat davon zusammen mit einem physiologisch annehmbaren Träger oder Exzipiens enthält.

**Patentansprüche für den Vertragsstaat: AT**

1. Verfahren zur Herstellung von Verbindungen der allgemeinen Formel (I)

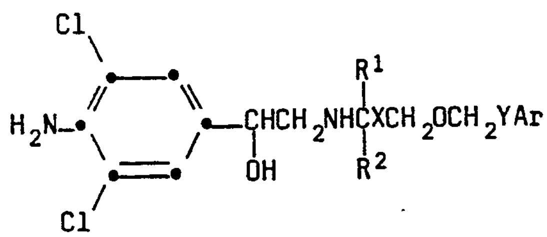

(I)

worin:

X für eine $C_{1-6}$-Alkylen-, $C_{2-6}$-Alkenylen- oder $C_{2-6}$-Alkinylenkette steht und

Y eine Bindung oder eine $C_{1-4}$-Alkylen, $C_{2-4}$-Alkenylen- oder $C_{2-4}$-Alkinylenkette bedeutet, mit der maßgabe, daß die Gesamtsumme der Kohlenstoffatome in X und Y nicht mehr als 8 ist;

Ar für eine Phenylgruppe steht, die gegebenenfalls durch ein oder mehrere Substituenten, ausgewählt aus Halogenatomen oder den Gruppen $C_{1-3}$-Alkyl, $C_{1-3}$-Alkoxy, —$(CH_2)_qOH$ (worin q eine ganze Zahl von 0 bis 3 bedeutet), —$NR^3R^4$ (worin $R^3$ und $R^4$ jeweils ein Wasserstoffatom oder eine $C_{1-4}$-Alkylgruppe bedeuten oder $NR^3R^4$ eine gesättigte heterocyclische Aminogruppe bildet, die 5 bis 7 Ringglieder hat und gegebenenfalls im Ring ein oder mehrere Atome, ausgewählt aus —O— oder —S—, oder eine Gruppe —NH— oder —$N(CH_3)$— enthält) oder —$NR^5COR^6$ (worin $R^5$ ein Wasserstoffatom oder eine $C_{1-4}$-Alkylgruppe und $R^6$ ein Wasserstoffatom oder eine $C_{1-4}$-Alkyl-, $C_{1-4}$-Alkoxy-, Phenyl- oder —$NR^3R^4$-Gruppe ist), substituiert ist, oder Ar eine Phenylgruppe ist, die durch eine Alkylendioxygruppe der Formel —$O(CH_2)_pO$— substituiert ist, wobei p den Wert 1 oder 2 hat;

$R^1$ und $R^2$ jeweils für ein Wasserstoffatom oder eine $C_{1-3}$-Alkylgruppe mit der Maßgabe stehen, daß die Gesamtsumme der Kohlenstoffatome in $R^1$ und $R^2$ nicht mehr als 4 ist,

und der physiologisch annehmbaren Salze und Solvate davon, dadurch gekennzeichnet, daß man

(1a) zur Herstellung von Verbindungen der Formel (I), bei denen $R^1$ ein Wasserstoffatom ist, ein Amin der allgemeinen Formel (II)

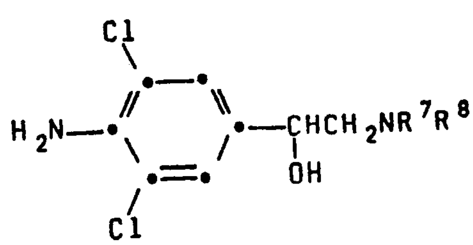

(II)

(worin $R^7$ ein Wasserstoffatom oder eine Schutzgruppe ist und $R^8$ für ein Wasserstoffatom steht) mit einem Alkylierungsmittel der allgemeinen Formel (III)

$$\underset{\substack{|\\R^2}}{LCH}XCH_2OCH_2YAr \qquad (III)$$

(worin L eine Austrittsgruppe ist) alkyliert und anschließend erforderlichenfalls irgendwelche vorhandenen Schutzgruppen entfernt oder daß man

(1b) zur Herstellung von Verbindungen der Formel (I), bei denen $R^1$ ein Wasserstoffatom ist, ein Amin der allgemeinen Formel (II), bei dem $R^7$ ein Wasserstoffatom oder eine Schutzgruppe ist und $R^8$ ein Wasserstoffatom oder eine in dieses unter Reaktionsbedingungen umwandelbare Gruppe ist, mit einer Verbindung der allgemeinen Formel (IV)

$$R^2COXCH_2OCH_2YAr \qquad (IV)$$

in Gegenwart eines Reduktionsmittels alkyliert und erforderlichenfalls irgendwelche vorhandenen Schutzgruppen entfernt oder daß man

(2) ein Zwischenprodukt der allgemeinen Formel (VI)

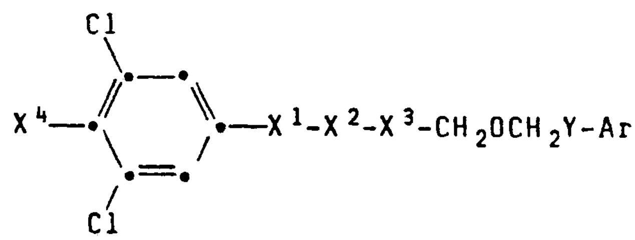

(VI)

worin

$X^1$ für —CH(OH)— oder eine in diese Gruppe durch Reduktion umwandelbare Gruppe steht,

$X^2$ für $CH_2NR^7$— oder eine in diese Gruppe durch Reduktion umwandelbare Gruppe steht,

$X^3$ für $—CR^1R^2X—$ oder eine in diese Gruppe durch Reduktion umwandelbare Gruppe steht,

$X^4$ für $—NH_2$ oder eine in diese Gruppe durch Reduktion umwandelbare Gruppe steht, Y wie in Anspruch 1 definiert ist oder eine Gruppe ist, die durch Reduktion in eine solche Gruppe umwandelbar ist, und Ar wie in Anspruch 1 definiert ist oder eine in eine solche Gruppe durch Reduktion umwandelbare Gruppe ist,

mindestens eine von $X^1$, $X^2$, $X^3$, $X^4$, Y und Ar eine reduzierbare Gurppe enthält, reduziert und anschließend erforderlichenfalls irgendwelche vorhandenen Schutzgruppen entfernt und daß man

gewünschtenfalls die resultierende Verbindung der allgemeinen Formel (I) oder ein Salz davon in ein physiologisch annehmbares Salz oder Solvat davon umwandelt.

2. Verfahren nach Anspruch 1 zur Herstellung von Verbindung, bei denen die durch X angegebene Kette für $—(CH_2)_2—$, $—(CH_2)_3—$, $—(CH_2)_4—$, $—(CH_2)_5—$, $—(CH_2)_6—$, $—CH_2C{\equiv}C—$, $—(CH_2)_2CH{=}CH—$, $—(CH_2)_2C{\equiv}C—$, $—CH{=}CHCH_2—$, $—CH{=}CH(CH_2)_2—$ oder $—CH_2C{=}CCH_2—$ und die durch Y angegebene Kette für $—CH_2—$, $—(CH_2)_2—$, $—(CH_2)_3—$, $—(CH_2)_4—$, $—CH{=}CH—$, $—C{\equiv}C—$, $—CH_2CH{=}CH—$ oder $—CH_2C{\equiv}C—$.

3. Verfahren nach Anspruch 1 zur Herstellung von Verbindungen, bei denen X für eine $C_{2-6}$-Alkylen- oder $C_{2-6}$-Alkinylenkette steht und Y für eine $C_{1-4}$-Alkylenkette steht.

4. Verfahren nach einem der Ansprüche 1 bis 3 zur Herstellung von Verbindungen, bei denen $R^1$ und $R^2$ unabhängig voneinander ein Wasserstoffatom oder eine Methylgruppe darstellen.

5. Verfahren nach einem der Ansprüche 1 bis 3 zur Herstellung von Verbindungen, bei denen $R^1$ und $R^2$ beide Wasserstoffatome sind oder $R^1$ ein Wasserstoffatom ist und $R^2$ eine $C_{1-3}$-Alkylgruppe ist.

6. Verfahren nach einem der Ansprüche 1 bis 5 zur Herstellung von Verbindungen, bei denen Ar eine unsubstituierte Phenylgruppe ist oder eine Phenylgruppe ist, die ein, zwei oder drei Substituenten, ausgewählt aus Brom, Iod, Chlor, Fluor, Methyl, Ethyl, Methoxy, Ethoxy, Amino, Methylamino, Ethylamino, Dimethylamino, Diethylamino, Morpholino, Piperidino, Piperazino, N-Methylpiperazino, $—NHCOR^6$ (worin $R^6$ für Wasserstoff, $C_{1-4}$-Alkyl, $C_{1-4}$-Alkoxy, Phenyl oder Amino steht), Hydroxyl, $—CH_2OH$ oder $—(CH_2)_2OH$, aufweist.

7. Verfahren nach Anspruch 1 zur Herstellung von Verbindungen der allgemeinen Formel (Ia)

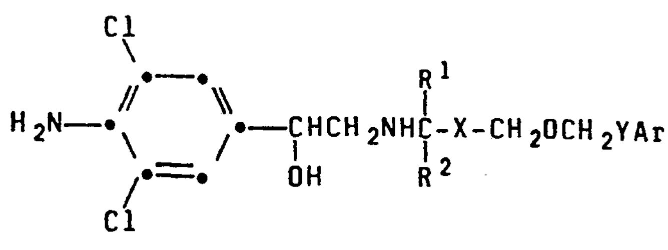

(Ia)

worin

X für eine $C_{3-4}$-Alkylen- oder $C_3$-Alkinylenkette steht;

Y für eine $C_{1-3}$-Alkylenkette steht;

$R^1$ und $R^2$ jeweils für Wasserstoff oder Methyl stehen; und

Ar eine Phenylgruppe bedeutet, die gegebenenfalls durch ein Fluoratom, eine Gruppe, ausgewählt aus Amino, $C_{1-3}$-Alkyl, $C_{1-3}$-Alkoxy, Hydroxy-$C_{1-2}$-alkyl, Morpholino, Hydroxy oder —$NHCOR^6$, wobei $R^6$ für $C_{1-3}$-Alkyl steht, substituiert ist, oder Ar eine Phenylgruppe ist, die durch Hydroxylgruppen in 3- und 5-Positionen substituiert ist,
und die physiologisch annehmbaren Salze und Solvate davon.

8. Verfahren nach Anspruch 7 zur Herstellung von Verbindungen, bei denen Ar eine Phenylgruppe ist, die gegebenenfalls einen Substituenten in 4-Position, der eine Amino-, —NH-Acetyl- oder Morpholinogruppe ist, enthält.

9. Verfahren nach Anspruch 1 zur Herstellung der Verbindungen:

4-Amino-3,5-dichlor-α-[[[6-[2-[4-(4-morpholinyl)-phenyl]-ethoxy]-hexyl]-amino]-methyl]-benzolmethanol;

N-[4-[2-[[6-[[2-(4-Amino-3,5-dichlorophenyl)-2-hydroxyethyl]-amino]-hexyl]-oxy]-ethyl]-phenyl]-acetamid;

4-Amino-3,5-dichlor-α-[[[6-[2-(4-aminophenyl)-ethoxy]-hexyl]-amino]-methyl]-benzolmethanol;

4-Amino-3,5-dichlor-α-[[[5-(2-phenylethoxy)-pentyl]-amino]-methyl]-benzolmethanol;

4-Amino-3,5-dichlor-α-[[[6-(2-phenylethoxy)-hexyl]-amino]-methyl]-benzolmethanol;

4-Amino-3,5-dichlor-α-[[[1-methyl-5-(2-phenylethoxy)-pentyl]-amino]-methyl]-benzolmethanol;

4-Amino-3,5-dichlor-α-[[[5-(2-phenylethoxy)-3-pentinyl]-amino]-methyl]-benzolmethanol;

[3-[[5-[[2-(4-Amino-3,5-dichlorphenyl)-2-hydroxyethyl]-amino]-pentyl]-oxy]-propyl]-1,3-benzoldiol
und die physiogisch annehmbaren Salze und Solvate davon.

**Revendications pour les Etats contractants: BE CH DE FR IT LI LU NL SE**

1. Composés répondant à la formule générale (I):

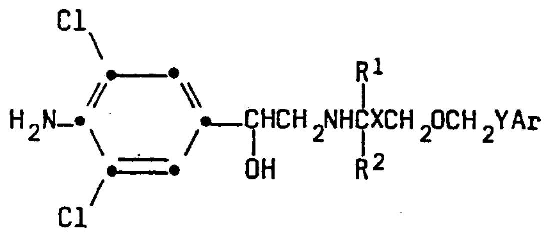

(I)

dans laquelle;

X représente une chaîne alkylène en $C_{1-6}$, alcénylène en $C_{2-6}$ ou alkynylène en $C_{2-6}$ et

Y représente une liaison ou une chaîne alkylène en $C_{1-4}$, alcénylène en $C_{2-4}$ ou alkynylène en $C_{2-4}$ à la condition que le total des atomes de carbone dans X et Y ne soit pas supérieur à 8; Ar représente un radical phényle facultativement substitué par un ou plusieurs substituant(s) choisi(s) parmi les atomes d'halogène ou les radicaux alkyle en $C_{1-3}$, alcoxy en $C_{1-3}$, —$(CH_2)_qOH$ (dans laquelle q est un nombre entier de 0 à 3), —$NR^3R^4$ (dans lequel $R^3$ et $R^4$ représentent chacun un atome d'hydrogène ou un radical alkyle en $C_{1-4}$, ou bien $NR^3R^4$ forme un groupe amino hétérocyclique saturé comportant 5 à 7 éléments de cycle et contenant facultativement dans le cycle un ou plusieurs atome(s) —O— ou —S— ou un groupe —NH— ou —$N(CH_3)$— ou —$NR^5COR^6$ (dans lequel $R^5$ représente un atome d'hydrogène ou un radical alkyle en $C_{1-4}$, et $R^6$ représente un atome d'hydrogène ou un radical alkyle en $C_{1-4}$, un radical alcoxy en $C_{1-4}$, phényle ou —$NR^3R^4$), ou bien Ar est un radical phényle substitué par un groupe alkylènedioxy de formule —$O(CH_2)_pO$—, dans lequel p est 1 ou 2;

$R^1$ et $R^2$ représentent chacun un atome d'hydrogène ou un radical alkyle en $C_{1-3}$ à la condition que le total d'atomes de carbone dans $R^1$ et $R^2$ ne dépasse pas 4; et leurs sels physiologiquement acceptables et leurs produits solvatisés.

2. Composés selon la revendication 1, dans lesquels la chaîne X représente: —$(CH_2)_2$—, —$(CH_2)_3$—, —$(CH_2)_4$—, —$(CH_2)_5$—, —$(CH_2)_6$—, —$CH_2C{\equiv}C$—, —$(CH_2)_2CH{=}CH$—, —$(CH_2)_2C{\equiv}C$—, —$CH{=}CHCH_2$—, —$CH{=}CH(CH_2)_2$—, ou —$CH_2C{\equiv}CCH_2$— et la chaîne Y est —$CH_2$—, —$(CH_2)_2$—, —$(CH_2)_3$—, —$(CH_2)_4$—, —$CH{=}CH$—, —$C{\equiv}C$—, —$CH_2CH{=}CH$—, ou —$CH_2C{\equiv}C$—.

3. Composés selon la revendication 1, dans lesquels X est une chaîne alkylène en $C_{2-6}$ ou alkynylène en $C_{2-6}$ et Y est un chaîne alkylène en $C_{1-4}$.

4. Composés selon l'une quelconque des revendications 1 à 3, dans lesquels $R^1$ et $R^2$ représentent indépendamment un atome d'hydrogène ou un radical méthyle.

5. Composés selon l'une quelconque des revendications 1 à 4, dans lesquels Ar est un radical phényle non substitue ou un radical phényle portant un, deux ou trois substituants choisis parmi les atomes de brome, d'iode, de chlore ou de fluor et les radicaux méthyle, éthyle, méthoxy, éthoxy, amino, méthylamino, éthylamino, diméthylamino, diéthylamino, morpholino, piperidino, piperazino, N-méthylpiperazino,

—$NHCOR^6$ (dans lequel $R^6$ est un atome d'hydrogène, n radical alkyle en $C_{1-4}$, alcoxy en $C_{1-4}$, phényle ou amino), hydroxy, —$CH_2OH$ ou —$(CH_2)_2OH$.

6. Composés répondant à la formule générale (1a):

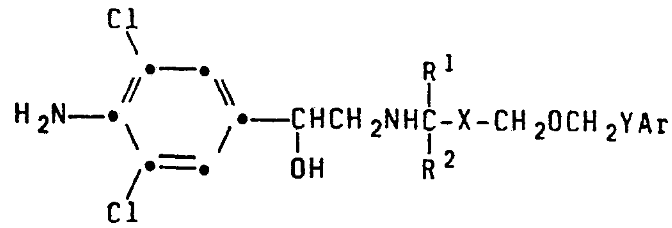

(Ia)

dans laquelle

X représente un chaîne alkylène en $C_{3-4}$ ou alkynylène en $C_3$;

Y représente une chaîne alkylène en $C_{1-3}$;

$R^1$ et $R^2$ représentent chacun un atome d'hydrogène ou un radical méthyle; et

Ar représente un radical phényle facultativement substitué par un atome de fluor, un groupe amino, alkyle en $C_{1-3}$, alcoxy en $C_{1-3}$, hydroxyalkyle en $C_{1-2}$, morpholino, hydroxy ou —$NHCOR^6$ dans lequel $R^6$ est un radical alkyle en $C_{1-3}$ ou Ar est un radical phényle substitué par des groupes hydroxyle en positions 3 et 5; et leurs sels et produits solvatisés physiologiquement acceptables.

7. Composés selon la revendication 6, dans lesquels Ar est un radical phényle contenant facultativement un substituant en position 4 qui est un radical amino, —NHAcétyle ou morpholino.

8. Composés:

4-amino-3,5-dichloro-α-[[[6-[2-[4-(4-morpholinyl)phényl]éthoxy]hexyl]amino]méthyl]benzène-méthanol;

N-[4-[2-[[6-[[2-(4-amino-3,5-dichlorophényl)-2-hydroxyéthyl]amino]hexyl]oxy]éthyl]phényl]acétamide;

4-amino-3,5-dichloro-α-[[[6-[2-(4-aminophényl)éthoxy]hexyl]amino]methyl]benzèneméthanol;

4-amino-3,5-dichloro-α-[[[5-(2-phényléthoxy]pentyl]amino]méthyl]benzèneméthanol;

4-amino-3,5-dichloro-α-[[[6-(2-phényléthoxy)hexyl]amino]méthyl]benzèneméthanol;

4-amino-3,5-dichloro-α-[[[1-méthyl-5-(2-phényléthoxy)pentyl]amino]méthyl]benzèneméthanol;

4-amino-3,5-dichloro-α-[[[5-(2-phényléthoxy)-3-pentynyl]amino]méthyl]benzèneméthanol;

[3-[[5-[[2-(4-amino-3,5-dichlorophényl)-2-hydroxyéthyl]amino]pently]oxy]propyl]-1,3-benzènediol;

et leurs sels et produits solvatisés physiologiquement acceptables.

9. Procédé de préparation de composés selon l'une quelconque des revendications 1 à 8 ou de leurs sels ou produits solvatisés physiologiquement acceptables, qui consiste:

(1a) pour la préparation de composés de formule (I) dans laquelle $R^1$ est un atome d'hydrogène, à alkyler une amine de formule générale (II):

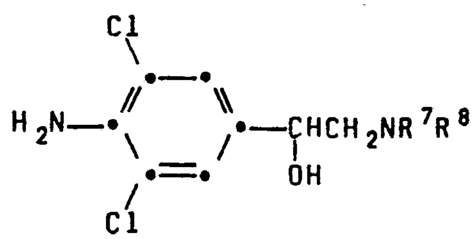

(II)

(dans laquelle $R^7$ est un atome d'hydrogène ou un groupe protecteur et $R^8$ est un atome d'hydrogène) avec un agent alkylant de formule générale (III):

$$\underset{\displaystyle R^2}{\underset{|}{L}}CHXCH_2OCH_2YAr \qquad (III)$$

(dans laquelle L est un groupe partant), puis à enlever, si nécessaire, les groupes protecteurs présents; ou

(Ib) pour la préparation de composés de formule (I) dans laquelle $R^1$ est un atome d'hydrogène, à alkyler une amine de formule générale (II) dans laquelle $R^7$ est un atome d'hydrogène ou un groupe protecteur et $R^8$ est un atome d'hydrogène ou un groupe convertible en celui-ci dans les conditions de la réaction, avec un composé de formule générale (IV):

$$R^2COXCH_2OCH_2YAr \qquad (IV)$$

en présence d'un agent réducteur puis, si nécessaire, à enlever les éventuels groupes protecteurs; ou

(2) à réduire un intermédiaire de formule générale (VI)

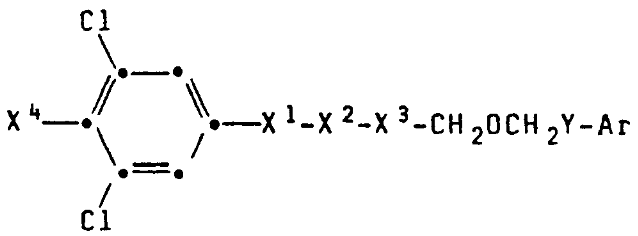

(VI)

dans laquelle;

$X^1$ représente —CH(OH)— ou un groupe convertible en celui-ci par réduction,

$X^2$ représente —$CH_2NR^7$— ou un groupe convertible en celui-ci par réduction,

$X^3$ représente —$CR^1R^2X$— ou un groupe convertible en celui-ci par réduction, $X^4$ représente —$NH_2$ ou un groupe convertible en celui-ci par réduction,

Y est tel que défini dans la revendication 1 ou un groupe convertible en celui-ci par réduction et

Ar est tel que défini dans la revendication 1 ou un groupe convertible en celui-ci par réduction,

au moins l'un des radicaux $X^1$, $X^2$, $X^3$, $X^4$, Y et Ar contenant un groupe réductible, qu'on fait suivre si nécessaire de l'enlèvement d'éventuels groupes protecteurs; et

à convertir, si on le désire, le composé résultant de formule générale (I) ou un sel de celui-ci en un sel physiologiquement acceptable ou un produit solvatisé.

10. Composition pharmaceutique comprenant au moins un composé de formule générale (I) tel que défini dans l'une quelconque des revendications 1 à 8 ou son sel ou produit solvatisé physiologiquement acceptable avec un véhicule ou excipient physiologiquement acceptable.

**Revendications pour l'Etat contractant: AT**

1. Procédé de préparation de composés de formule générale (I):

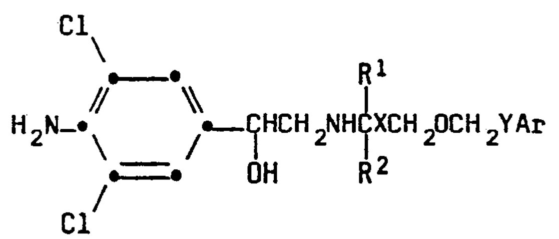

(I)

dans laquelle;

X représente une chaîne alkylène en $C_{1-6}$, alcénylène en $C_{2-6}$ ou alkynylène en $C_{2-6}$ et

Y représente une liaison ou une chaîne alkylène en $C_{1-4}$, alcénylène en $C_{2-4}$ ou alkynylène en $C_{2-4}$ à la condition que le total des atomes de carbone dans X et Y ne soit pas supérieur à 8; Ar représente un radical phényle facultativement substitué par un ou plusieurs substituant(s) choisi(s) parmi les atomes d'halogène ou les radicaux alkyle en $C_{1-3}$, alcoxy en $C_{1-3}$, —$(CH_2)_qOH$ (dans laquelle q est un nombre entier de 0 à 3), —$NR^3R^4$ (dans lequel $R^3$ et $R^4$ représentent chacun un atome d'hydrogène ou un radical alkyle en $C_{1-4}$, ou bien $NR^3R^4$ forme un groupe amino hétérocyclique saturé comportant 5 à 7 éléments de noyau et contenant facultativement dans le noyau un ou plusieurs atome(s) —O— ou —S— ou un groupe —NH— ou —$N(CH_3)$— ou —$NR^5COR^6$ (dans lequel $R^5$ représente un atome d'hydrogène ou un radical alkyle en $C_{1-4}$, et $R^6$ représente un atome d'hydrogène ou un radical alkyle en $C_{1-4}$, un radical alcoxy en $C_{1-4}$, phényle ou —$NR^3R^4$), ou bien Ar est un radical phényle substitué par un groupe alkylènedioxy de formule —$O(CH_2)_pO$—, dans lequel p est 1 ou 2;

$R^1$ et $R^2$ représentent chacun un atome d'hydrogène ou un radical alkyle en $C_{1-3}$ à la condition que le total d'atomes de carbone dans $R^1$ et $R^2$ ne dépasse pas 4; et leurs sels physiologiquement acceptables et leurs produits solvatisés qui consiste:

(1a) pour la préparation de composés de formule (I) dans laquelle $R^1$ est un atome d'hydrogène, à alkyler une amine de formule générale (II):

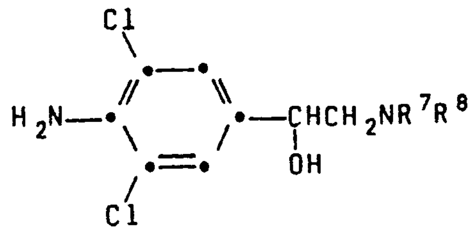 (II)

(dans laquelle $R^7$ est un atome d'hydrogène ou un groupe protecteur et $R^8$ est un atome d'hydrogène) avec un agent alkylant de formule générale (III):

$$\underset{\displaystyle R^2}{\underset{|}{LCH}}XCH_2OCH_2YAr \qquad (III)$$

(dans laquelle L est un groupe partant), puis à enlever, si nécessaire, les groupes protecteurs présents; ou

(Ib) pour la préparation de composés de formule (I) dans laquelle $R^1$ est un atome d'hydrogène, à alkyler une amine de formule générale (II) dans laquelle $R^7$ est un atome d'hydrogène ou un groupe protecteur et $R^8$ est un atome d'hydrogène ou un groupe convertible en celui-ci dans les conditions de la réaction, avec un composé de formule générale (IV):

$$R^2COXCH_2OCH_2YAr \qquad (IV)$$

en présence d'un agent réducteur puis, si nécessaire, à enlever les éventuels groupes protecteurs; ou

(2) à réduire un intermédiaire de formule générale (VI)

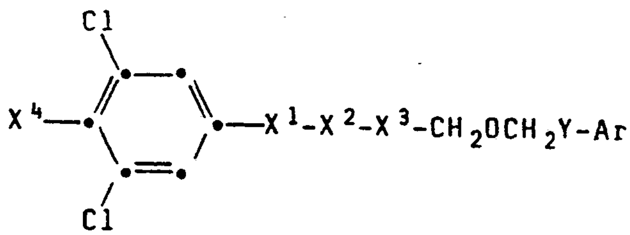 (VI)

dans laquelle;

$X^1$ représente —CH(OH)— ou un groupe convertible en celui-ci par réduction,

$X^2$ représente $—CH_2NR^7—$ ou un groupe convertible en celui-ci par réduction,

$X^3$ représente $—CR^1R^2X—$ ou un groupe convertible en celui-ci par réduction, $X^4$ représente $—NH_2$ ou un groupe convertible en celui-ci par réduction,

Y est tel que défini dans la revendication 1 ou un groupe convertible en celui-ci par réduction et Ar est tel que défini dans la revendication 1 ou un groupe convertible en celui-ci par réduction,

au moins l'un des radicaux $X^1$, $X^2$, $X^3$, $X^4$, Y et Ar contenant un groupe réductible, qu'on fait suivre si nécessaire de l'enlèvement d'éventuels groupes protecteurs; et

à convertir, si on le désire, le composé résultant de formule générale (I) ou un sel de celui-ci en sel physiologiquement acceptable ou un produit solvatisé.

2. Procédé selon la revendication 1, pour préparation de composés dans lesquels la chaîne X représente: $—(CH_2)_2—$, $—(CH_2)_3—$, $—(CH_2)_4—$, $—(CH_2)_5—$, $—(CH_2)_6—$, $—CH_2C{\equiv}C—$, $—(CH_2)_2CH{=}CH—$, $—(CH_2)_2C{\equiv}C—$, $—CH{=}CHCH_2—$, $—CH{=}CH(CH_2)_2—$, ou $—CH_2C{\equiv}CCH_2—$ est la chaîne Y est $—CH_2—$, $—(CH_2)_2—$, $—(CH_2)_3—$, $—(CH_2)_4—$, $—CH{=}CH—$, $—C{\equiv}C—$, $—CH_2CH{=}CH—$, ou $—CH_2C{\equiv}C—$.

3. Procédé selon la revendication 1, pour la préparation de composés dans lesquels X repésente une chaîne alkylène en $C_{2-6}$ ou alkynylene en $C_{2-6}$ et Y représente une chaîne alkylene en $C_{1-4}$.

4. Procédé selon l'une quelconque des revendications 1 à 3 pour la production de composés dans lesquels $R^1$ et $R^2$ représentent indépendamment un atome d'hydrogène ou un radical méthyle.

5. Procédé selon l'une quelconque des revendications 1 à 3, pour la préparation de composés dans lesquels $R^1$ et $R^2$ sont tous deux des atomes d'hydrogène ou $R^1$ est un atome d'hydrogène et $R^2$ est un radical alkyle en $C_{1-3}$.

6. Procédé selon l'une quelconque des revendications 1 à 5, pour la préparation de composés dans lesquels Ar est un radical phényle non substitué ou un radical phényle substitué par un, deux ou trois substituants choisis parmi les atomes de brome, iode, de chlore ou fluor et les radicaux méthyle, éthyle, méthoxy, éthoxy, amino, méthylamino, éthylamino, diméthylamino, diéthylamino, morpholino, piperidino, piperazino, N-méthylpiperazino, $—NHCOR^6$ (dans lequel $R^6$ est un atome d'hydrogène, ou un radical alkyle en $C_{1-4}$, alcoxy en $C_{1-4}$, phényle ou amino), hydroxyle, $—CH_2OH$ ou $—(CH_2)_2OH$.

7. Procédé selon la revendication 1, pour la préparation de composés de formule générale (Ia):

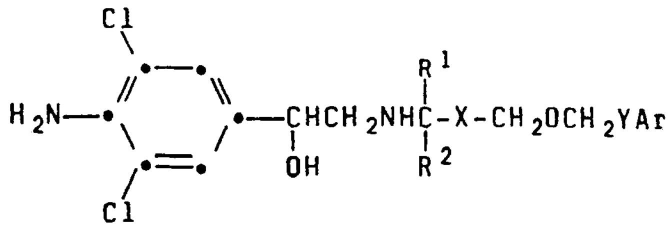

(Ia)

dans laquelle

X représente un chaîne alkylène en $C_{3-4}$ ou alkynylène en $C_3$;

Y représente une chaîne alkylène en $C_{1-3}$;

$R^1$ et $R^2$ représentent chacun un atome d'hydrogène ou un radical méthyle; et

Ar représente un radical phényle facultativement substitué par un atome de fluor, un groupe amino, alkyle en $C_{1-3}$, alcoxy en $C_{1-3}$, hydroxyalkyle en $C_{1-2}$, morpholino, hydroxy ou —NHCOR$^6$ dans lequel R$^6$ est un radical alkyle en $C_{1-3}$ ou Ar est un radical phényle substitué par des groupes hydroxyle en positions 3 et 5; et leurs sels et produits solvatisés physiologiquement acceptables.

8. Procédé selon la revendication 7, pour la préparation de composés dans lesquels Ar est un radical phényle contenant facultativement en position 4 un substituant qui est un radical amino, —NHAcétyle ou morpholino.

9. Procédé selon la revendication 1, pour la préparation de composés:

4-amino-3,5-dichloro-α-[[[6-[2-[4-(4-morpholinyl)phényl]éthoxy]hexyl]amino]méthyl]benzène-méthanol;

N-[4-[2-[[6-[[2-(4-amino-3,5-dichlorophényl)-2-hydroxyéthyl]amino]hexyl]oxy]éthyl]phényl]acétamide;

4-amino-3,5-dichloro-α-[[[6-[2-(4-aminophényl)éthoxy]hexyl]amino]methyl]benzèneméthanol;

4-amino-3,5-dichloro-α-[[[5-(2-phényléthoxy]pentyl]amino]méthyl]benzèneméthanol;

4-amino-3,5-dichloro-α-[[[6-(2-phényléthoxy)hexyl]amino]méthyl]benzèneméthanol;

4-amino-3,5-dichloro-α-[[[1-méthyl-5-(2-phényléthoxy)pentyl]amino]méthyl]benzèneméthanol;

4-amino-3,5-dichloro-α-[[[5-(2-phényléthoxy)-3-pentynyl]amino]méthyl]benzèneméthanol;

[3-[[5-[[2-(4-amino-3,5-dichlorophényl)-2-hydroxyéthyl]amino]pentyl]oxy]propyl]-1,3-benzènediol;

et les sels et produits solvatisés physiologiquement acceptables de ces derniers.